(19) Europäisches Patentamt
European Patent Office
Office européen des brevets

(11) **EP 3 677 171 B1**

(12) **EUROPEAN PATENT SPECIFICATION**

(45) Date of publication and mention
of the grant of the patent:
**01.11.2023 Bulletin 2023/44**

(21) Application number: **20150550.0**

(22) Date of filing: **07.01.2020**

(51) International Patent Classification (IPC):
**A61B 5/00** *(2006.01)*          **A61B 5/024** *(2006.01)*
**A61B 5/11** *(2006.01)*

(52) Cooperative Patent Classification (CPC):
**A61B 5/4815; A61B 5/02405; A61B 5/1118;
A61B 5/681; A61B 5/741;** A61B 2503/10

(54) **A METHOD AND APPARATUS FOR DETERMINING SLEEP NEED AND SLEEP PRESSURE BASED ON PHYSIOLOGICAL DATA**

VERFAHREN UND VORRICHTUNG ZUR BESTIMMUNG DES SCHLAFBEDÜRFNISSES UND DES SCHLAFDRUCKS AUF BASIS PHYSIOLOGISCHER DATEN

PROCÉDÉ ET APPAREIL PERMETTANT DE DÉTERMINER UN BESOIN EN SOMMEIL ET LA PRESSION DU SOMMEIL SUR LA BASE DE DONNÉES PHYSIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.01.2019 US 201962789156 P**

(43) Date of publication of application:
**08.07.2020 Bulletin 2020/28**

(73) Proprietor: **Firstbeat Analytics OY
40100 Jyväskylä (FI)**

(72) Inventors:
• **MYLLYMÄKI, Tero
40100 JYVÄSKYLÄ (FI)**
• **HUJANEN, Wille
40100 JYVÄSKYLÄ (FI)**
• **SAALASTI, Sami
40100 JYVÄSKYLÄ (FI)**
• **RUHANEN, Tuukka
40100 JYVÄSKYLÄ (FI)**
• **LUUKKO, Perttu
40100 JYVÄSKYLÄ (FI)**
• **TOIVONEN, Johanna
40100 JYVÄSKYLÄ (FI)**

(74) Representative: **Kespat Oy
Vasarakatu 1
40320 Jyväskylä (FI)**

(56) References cited:
**US-A1- 2011 230 790    US-A1- 2015 116 117
US-A1- 2016 374 567    US-A1- 2017 132 946
US-A1- 2018 085 549**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD**

The present method and apparatus relate to determining an individualized need for sleep and real-time indication of sleep pressure from physiological data including at least heart rate data.

**BACKGROUND**

Key factors of athletic performance have been shown to be negatively affected by insufficient sleep or sleep deprivation. For example, physical abilities, such as speed and endurance, can be negatively impacted, as can aspects of neurocognitive function such as attention and memory. Likewise, it can have major impacts on physical health, such as risk of illness and risk of injury, or the ease with which a person can maintain a desired body weight and composition. It has been suggested that athletes having a high training load need comparably more sleep than non-athletes to ensure adequate physiological and psychological recovery caused by training. Research also supports the idea that athletes may benefit from sleeping more than generally recommended for facilitating recovery and maintaining peak performance. This may additionally be compounded with other factors, as well; for example, competitive athletes are often under a high level of stress, something which can significantly affect sleep need and sleep quality on its own. (Factors like stress can also be significant for non-athletes, as well.)

However, it has historically been difficult to determine, even on a demographic basis, how much sleep any individual person is likely to need at any given time. Not only is this likely to vary widely from person to person but is likely to vary substantially over the course of the person's lifetime; for example, often, a person will need less sleep as they age. Any other factors which may affect their sleep needs may affect their sleep needs in a manner wildly different from others, with some people being very adversely affected by certain stimuli and other people being unaffected. This, of course, does not take into account the person's activities or activity level, such as their athletic activity level, at all, which may further complicate matters.

As such, most sleep research has been able to produce extremely loose and general guidelines, at best, for how much sleep may be necessary for a given person at a given time. For example, the National Sleep Foundation (NSF), a US-based nonprofit organization that promotes and sponsors sleep research, has published recommendations stating that, for most people of a certain age, a relatively broad range of sleep times are "recommended" or "may be appropriate," which may vary by up to five hours. For example, it is noted that for a young adult, aged 18-25 years, it may be appropriate for them to receive anywhere from 6 to 11 hours of sleep at night, with 7-9 being generally recommended. As the minimum potentially appropriate value in this range is almost half of the maximum potentially appropriate value, young adults are given little guidance as to how much sleep they might actually need. These values are, of course, an approximation, which may be heavily impacted based on key factors such as lifestyle and health, daily stress levels, "sleep debt," use of drugs such as caffeine, alcohol, nicotine, or prescription drugs, alarm clocks, and external lights (including streetlights, electronic displays, and even power lights and the like on electronic devices) which may interfere with that person's circadian rhythms or natural sleep-wake cycle.

Such research readily admits that research cannot pinpoint the exact amount of sleep needed for people of different ages. People interested in enhancing their sleep schedule can use the results of current research only as a starting point or rule-of-thumb. Using those rules of thumb as a starting point, visitors to the NSF site may be asked highly subjective questions such as "Are you productive, healthy and happy on seven hours of sleep? Or does it take you nine hours of quality sleep to get you into high gear?", "Do you have health issues such as being overweight? Are you at risk for any disease?", "Are you experiencing sleep problems?", "Do you depend on caffeine to get you through the day?" or "Do you feel sleepy when driving?" Of course, the answers to such questions may vary significantly for each visitor, who may, for example, have entirely different standards as to how much caffeine use constitutes a "dependency," and visitors with significantly different opinions from the test creators may be classified incorrectly, such that too much or too little sleep is recommended.

Patent US 8,468,115 presents systems for treating cyclical behaviors like sleeping trough colleting behavioral data and giving recommendations.

Patent US 10,325,514 presents methods for setting up and tracking sleep consistency goals of users. For example, a biometric monitoring device with a sleep data logger calculates a target bedtime based on a schedule waketime and sleep efficiency.

Application US 2016374567 A1 discloses a system that uses continuous tracking of sleep activity and heart rate activity to evaluate heart rate variability immediately before transitioning to an awake state, e.g., at the end of the last phase of deep sleep. In particular, a wearable, continuous physiological monitoring system includes one or more sensors to detect sleep states, the transitions between sleep states, and the transitions from a sleep state to an awake state for a user. This information can be used in conjunction with continuously monitored heart rate data to calculate heart rate variability of the user at the end of the last phase of sleep preceding the user waking up. By using the history of heart rate data in conjunction with sleep activity in this manner, an accurate and consistent recovery score can be calculated based on heart rate variability.

Application US 2018085549 A1 discloses a method, apparatus, and computer program product for providing a dynamic wake-up alert. A user's sleep recovery need is determined based on a variety of factors, including but not limited to, mental stress level, physical activity, the individual's sleep history record, and/or data relating to circadian rhythms, recommended sleep times, patterns and cycles. Real-time sleep data, such as that detected by a sleep data detection device, is monitored to determine a real-time gained recovery. Biological, physiological, and/or neurological data relating to the quality of sleep, and/or the amount of sleep obtained is used to calculate the real-time gained sleep recovery of the user and compare the gained recovery to the sleep recovery need. Once the sleep recovery need is satisfied, a dynamic wake-up alert is provided via a user interface.

Application US 2015116117 A1 discloses a system for providing a sleep recommendation includes an apparatus for providing a sleep recommendation. The apparatus includes a preferred sleep determination module that determines a preferred sleep duration. The apparatus also includes a sleep debt module that creates and updates a sleep debt based on the preferred sleep duration and an actual sleep duration. In addition, the apparatus includes a sleep recommendation module that provides a recommended sleep duration based on the sleep debt.

It would be of great benefit to an individual to know, with a greater level of accuracy, how much sleep they actually need, and when they should go to bed, at an individually tailored level. Providing an individual with this information, i.e. an indication of recommended sleep duration for that person, would currently require a complicated and highly arbitrary balance of the various factors and activities which are consuming or producing energy for them in their daily life. This information would vary based on their daily actions.

## SUMMARY

The aim of this invention is to achieve a method and apparatus for determining an individualized need for sleep and real-time indication of sleep pressure from physiological data, including at least heart rate data and movement data. The aim is achieved by the features presented in claims.

The method is based on an empirically created modelling technique, where the baseline of sleep need is determined using background data, particularly age of a user. That is converted to a personal sleep need using at least stress and training data models, preferably also sleep history data. In certain exemplary embodiments, the modelling techniques used by the system may include the use of separate models of personal sleep need and baseline sleep need, with one or more of these models taking into account some or all of: stress and relaxation intensity, training load information (including one or more of short-term training load information, long-term training load information, and absolute weekly training load information), and sleep score information depicting sleep history.

The software may produce personal sleep need and/or sleep pressure values on periodic schedule, such as once or twice a day, which may instantly in any time be adjusted to instant values, when called by a child process. The adjustment takes place at least by instant body resource and elapsed time from last sleep, body resource being in terms of the physiological resources currently available to the user, as determined from the measurement of physiological data. In particular, body resource value may be analyzed from physiological data such as heart rate variability (HRV) data and motion data.

The selected empiric sleep need change functions converting baseline need to personal need include at least stress history and training history. The training history may consist of short-term training load (WTL), long term training load (MTL) and absolute weekly training load. Sleep history may also be used in said conversion.

A benefit of the above empiric approach, and the selection of the specific variables contemplated above, is that it may allow for accurate calculations to be completed using relatively small hardware resources. As such, the use of the empiric modelling techniques and specific selection of variables may offer specific tangible benefits on their own. Other benefits may be provided through the use of the calculation structure, where an initial sleep pressure value per time unit is obtained. The result may specifically be obtained by specific body resources correction and multiplication with elapsed time of awake/asleep.

Many of the examples and example calculations provided herein may make use of a particular experimental dataset used to develop and validate the applicant's sleep detection and sleep stage classification methodology. This dataset included 110 nights (780 hours) of expertly stage-categorized polysomnography sleep data from adult subjects. It also included heart rate data in the form of RR-intervals obtained from ECG measurement and was accompanied by movement data obtained using accelerometers. The separate correction functions were obtained by statistical analysis. Available software which may be used to replicate this includes, for example MATLAB® with Statistical Toolbox. "Movement data" may be contemplated to include movement data derived from one or more of a variety of sources; for example, it may be contemplated to have "movement data" be measured by GPS, accelerometer or a similar sensor.

According to an exemplary embodiment, a method and apparatus which may be used to determine a user's overall need for sleep and indicate the user's body's status regarding their need for sleep (their "sleep pressure") in real-time may be provided.

Preferably, it is selected from a set of feedback dialogues associated with predetermined sleep need and sleep pressure values, a feedback dialogue to be displayed to the user. The feedback dialogue is provided preferably automatically, wherein automatic provision of the feedback dialogue is provided at least one of the set of: a predetermined time before the recommended bedtime and a predetermined time before a start of the recommended bedtime range.

In an exemplary embodiment, such a method can be implemented in an embedded device having limited CPU and memory resources and having a host system. In one exemplary embodiment, the host system may make use of ETE and THA -software libraries (which may generally be referred to as "THA"), wherein an ETE library is a real-time heart rate analysis library software (host process), and a THA library is a training history analysis library. In an exemplary embodiment, THA-software (child process) may be called and executed temporarily to calculate the need for sleep value.

According to an exemplary embodiment, it may be contemplated to reduce the demand of hardware resources - particularly random-access memory (RAM) or dynamic memory in general - by selecting key variables suitably. In such an exemplary embodiment, the amount of resident memory that may be demanded may be very limited, and the system may be configured to store only characteristics of each exercise and each night's sleep. The information stored may include, for example, time stamp information regarding physical activity. It may further include TRIMP ("TRaining IMPulse") data, which may generally quantify training effect and may be based on criteria such as heart rate, heart rate reserve, and the duration that the user maintained a particular heart rate. The TRIMP data may be quantified or scaled in a variety of known ways, such as by the average heart rate per minute (TRIMP_avg), or by an exponential scaling factor to account for higher intensity training (TRIMP_exp). It may further include EPOC (excess post-exercise oxygen consumption) data, a measure of the user's increased rate of oxygen intake following strenuous activity, also called "afterburn." It may also include, for example, a sleep score result and so on.

In an exemplary embodiment, a calculation may use training history data for all kinds of exercise types (e.g. running, bicycling, rowing, gym exercises etc.). The calculation may analyze absolute training load and save results to internal memory, known as the Sleep Need value. This information may be available for a certain amount of time, such that the system maintains, for example, a 7 or a 28-day training history. The system may be configured to store and take into consideration a predefined range of training history data, or another (optionally customizable or otherwise dynamic) length of time such as may be desired. For example, a user that has suffered some major interruption in their training history, such as an injury, may wish to restart their progress after the injury so as not to receive biased results. An exemplary embodiment may take into consideration up to 365 days (1 year) of training history data.

It may generally be understood, from the literature on physical training, that the harder the training has been, the more the homeostasis of the body is disturbed. The more that the homeostasis has been disturbed, the greater the adaptations that can be created in the body and the improvements in physical condition that derive from the adaptations. Thus, in an exemplary embodiment, the variable that may be used to characterize training load may be a peak value regarding training effect, measured as a disturbance level of homeostasis. The disturbance in the level of homeostasis may be determined by measuring a user's physiological resources (i.e. physiological data generally relating to the user's ability to continue to perform) and obtaining physiological data characterizing those physiological resources, which may generally be referred to as "body resources" or "body resource values.". As a person with a greater fitness level needs greater training stimulus to disturb homeostasis, the training load can be scaled to each individual's capabilities in order to depict training effect.

According to an exemplary embodiment, a sleep score can be measured based on an analysis of heart rate variability (HRV) and, optionally, acceleration data. For acquiring HRV information, any electrocardiography sensors devices or sensors may be used, such as ECG or PPG-based devices or sensors, as desired.

In an exemplary embodiment, the "sleep need" and "sleep pressure" functions indicate an adequate amount of sleep for an individual person based on the person's age and based on a consideration of lifestyle factors including stress level, training load, sleep quality, and the balance between stress and recovery. The stress level, training load, and sleep period/quality data may be determined from measurement of a person's physiological resources. The physiological resources may be measured or estimated based on heart rate data, such as heart rate variability, or motion data measured using, for example, an accelerometer. In addition, the functions may indicate, in real-time, the body's need to rest, and may guide the user towards an appropriate time for going to bed.

An exemplary method for analyzing sleep may calculate a baseline sleep need value based on a user's age, and then may adjust the sleep need value based on the user's stress level data, training load data, and sleep period data. The method may then create a sleep pressure value based on the sleep need value. The sleep pressure value may be adjusted to account for the user's sleep and awake periods, the time elapsed from a previous night's sleep, and a user's body resources (i.e. the physiological resources currently available to that particular user), as determined from the measurement of physiological data. The physiological data may be continuously measured, and as a result the stress level data, training load data, sleep period data, and/or the body resource data may be continuously updated as well as the sleep need and sleep pressure values. The sleep need and sleep pressure values may change throughout the day, and a user may be presented with current values which accounts for recent data or measurements.

The calculated sleep pressure and sleep need value may be used to select one of various possible feedback dialogues which may present the user with information regarding their sleep need or sleep pressure, as well as provide recommendations based on those values. The feedback dialogues may recommend that a user reduce stress for the rest of the day or may recommend an ideal time to go to bed.

In another exemplary embodiment, a computer program product may implement the method for analyzing sleep. The computer program product may receive a user's age and physiological data as input, as measured from a sensor such as a heart rate sensor or an ECG. The program may then implement the method and may calculate a sleep need value and sleep pressure value. The program may create or choose appropriate feedback dialogues based on the calculated sleep need and sleep pressure values, and may present the feedback dialogues on the device which the program is executed on, or, alternatively, may send the feedback dialogues to an external device which then presents the feedback to the user.

In another exemplary embodiment, a device may be implemented to analyze sleep. The device may include a memory module, an input module, an accelerometer and/or a gyroscope, a heart rate sensor such as an ECG. The device may also include a processing unit configured to calculate a sleep need value and a sleep pressure value based on data read from the heart rate sensor and the accelerometer. The processor may then select a feedback dialogue which may be presented to the user using an output module, such as a screen or a speaker. The output module may also be an external device separate from the device which performs the analysis.

## BRIEF DESCRIPTION OF THE FIGURES

Advantages of embodiments of the present invention will be apparent from the following detailed description of the exemplary embodiments thereof, which description should be considered in conjunction with the accompanying drawings in which like numerals indicate like elements, in which:

Figure 1 is an exemplary process flow diagram describing an exemplary embodiment of an analysis process for sleep need and sleep pressure.

Figure 2a is another diagram describing an exemplary embodiment of an analysis process for sleep need and sleep pressure in more detail.

Figure 2b presents example of a set of values for calculation according to diagram in figure 2a

Figure 3a is an exemplary embodiment of a graph showing one of the factors and functions which may affect sleep need, specifically stress and relaxation intensity.

Figure 3b is an exemplary embodiment of a graph showing one of the factors and functions which may affect sleep need, specifically short-term training load.

Figure 3c is an exemplary embodiment of a graph showing one of the factors and functions which may affect sleep need, specifically long-term training load.

Figure 3d is an exemplary embodiment of a graph showing one of the factors and functions which may affect sleep need, specifically sleep score.

Figure 3e is an exemplary embodiment of a graph showing one of the factors and functions which may affect sleep need, specifically absolute weekly training load.

Figure 4 is an exemplary depiction of a set of example feedback which may indicate sleep need and sleep pressure on a wearable device.

Figure 5 is an exemplary embodiment of adjusted sleep score calculations based on sleep score and sleep quality information.

Figure 6 is an exemplary graph showing a change in recommended sleep duration based on RSI.

Figure 7 is an example of a hardware assembly.

## DETAILED DESCRIPTION

Aspects of the invention are disclosed in the following description and related drawings directed to specific embodiments of the invention. Alternate embodiments may be devised without departing from the scope of the invention. Additionally, well-known elements of exemplary embodiments of the invention will not be described in detail or will be omitted so as not to obscure the relevant details of the invention. Further, to facilitate an understanding of the description discussion of several terms used herein follows.

As used herein, the word "exemplary" means "serving as an example, instance or illustration." The embodiments described herein are not limiting, but rather are exemplary only. It should be understood that the described embodiments are not necessarily to be construed as preferred or advantageous over other embodiments. Moreover, the terms "embodiments of the invention", "embodiments" or "invention" do not require that all embodiments of the invention include the discussed feature, advantage or mode of operation.

Further, many embodiments are described in terms of sequences of actions to be performed by, for example, elements of a computing device. It will be recognized that various actions described herein can be performed by specific circuits (e.g., application specific integrated circuits (ASICs)), by program instructions being executed by one or more processors, or by a combination of both. Additionally, these sequences of actions described herein can be considered to be embodied entirely within any form of computer readable storage medium having stored therein a corresponding set of computer instructions that upon execution would cause an associated processor to perform the functionality described herein. Thus, the various aspects of the invention may be embodied in a number of different forms, all of which have been contemplated to be within the scope of the claimed subject matter. In addition, for each of the embodiments described herein, the corresponding form of any such embodiments may be described herein as, for example, "logic configured to" perform the described action.

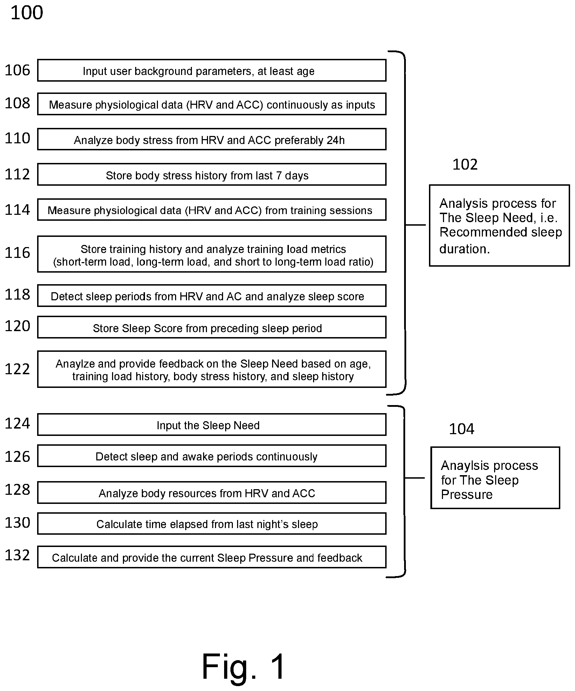

According to an exemplary embodiment, a method for determining sleep need and sleep pressure may include the steps shown in Figure 1.

In various exemplary embodiments, the calculation of sleep pressure 100 may generally include two steps, the calculation of sleep need 102, and the calculation of sleep pressure based on sleep need 104. These two steps may include a number of sub-steps.

A first part of this process, calculation of Sleep Need 102, may proceed as follows. In an exemplary embodiment, the calculation process for sleep need, i.e. recommended sleep duration for a person, may start from user background parameters, such as a default age-based value 106. An age-based default sleep need may be modified further by additional calculations. Predetermined constant values may be used, or, in an exemplary embodiment, a default value may be calculated, as follows.

$$\text{Sleep need in minutes (default)} = (-0.2015 \times \text{age} + 12.14) \times 60 \text{ (when age} < 21) \text{ OR} = (-0.0085 \times \text{age} + 8.18) \times 60 \text{ (when age} > 21).$$

The table below indicates exemplary default values based on age groups.

**Table 1: Exemplary constant default values, by age.**

| Age | Default value |
| --- | --- |
| 6-13 yrs | 10 hours |
| 14-17 yrs | 9 hours |
| 18-64 yrs | 8 hours |
| 65+ | 7.5 hours |

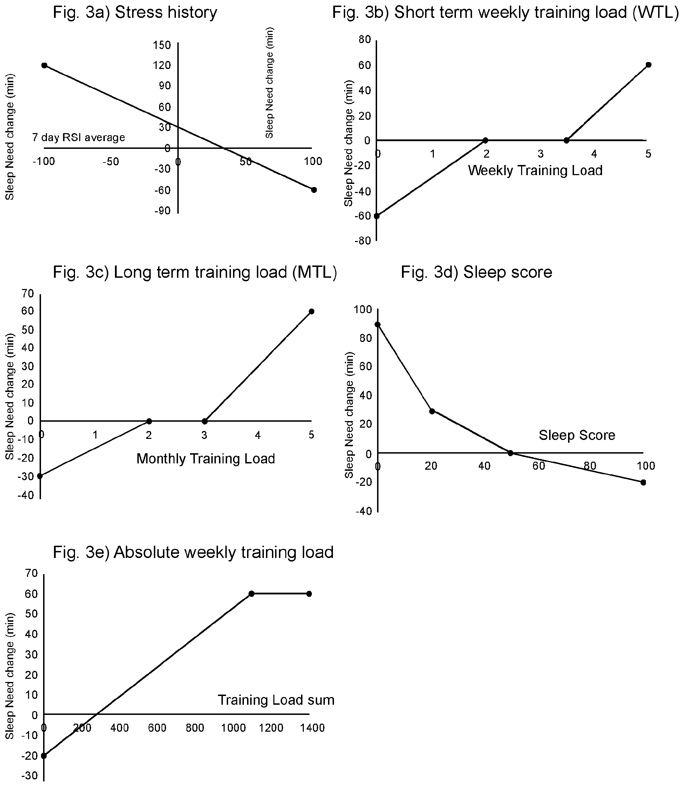

In an exemplary embodiment, the calculation process may further add and/or subtract the effects of other lifestyle and training related factors affecting the need for sleep. Figure 3 describes certain other factors that may affect the Sleep Need function. Those factors may take many forms such as linear or partially linear dependency between a lifestyle factor and the Sleep Need.

Still referring to exemplary Fig. 1, a next exemplary step may measure physiological data continuously as inputs 108. Data that may be used in order to characterize a person's physiological resources (or "physiological data" as used herein) may relate to measurements taken from a variety of sensors which measure a bodily function, such as heart rate. The physiological data may include heart rate variable data and/or motion data. A variety of devices or heart rate sensors may record the heart rate data, such as electrocardiography sensors as well as other devices such as ECG or PPG-based devices or sensors, as desired. Motion data may be recorded using similarly known devices that record motion data, such as accelerometers. The physiological data may be analyzed to determine a body stress level 110. The body stress level may be a compilation of physiological data over a period of time. In an exemplary embodiment, the body stress level may be calculated based on 24 hours of physiological data. In a next exemplary step, a body stress history may be calculated based on the body stress level. The body stress history may be a compilation of body stress levels over a longer period of time, such as over a period of 7 days.

Still referring to exemplary Fig. 1, the method may include another optional step of measuring physiological data during various training sessions 114. As a result, the method may store the physiological data related to the training sessions to create a training history 116. The training history may include training load metrics, such as short-term load, long-term load, and short to long-term load ration. In an exemplary embodiment, a calculation may use training history data for all kinds of exercise types (e.g. running, bicycling, rowing, gym exercises etc.). The calculation may analyze absolute training load and save it to internal memory. This information may be available for a certain amount of time, such that the system maintains, for example, a 7 or a 28-day training history. The system may be configured to store and take into consideration a predefined range of training history data, or another (optionally customizable or otherwise dynamic) length of time such as may be desired. For example, a user that has suffered some major interruption in their training history, such as an injury, may wish to restart their progress after the injury so as not to receive biased results. An exemplary embodiment may take into consideration up to 365 days (1 year) of training history data. Training history data may be stored as TRIMP ("TRaining IMPulse") data, which may generally quantify training effect and may be based on criteria such as heart rate, heart rate reserve, and the duration that the user maintained a particular heart rate. The TRIMP data may be quantified or scaled in a variety of known ways, such as by the average heart rate per minute (TRIMP_avg), or by an exponential scaling factor to account for higher intensity training (TRIMP_exp). It may further include EPOC (excess post-exercise oxygen consumption) data, a measure of the user's increased rate of oxygen intake following strenuous activity, also called "afterburn".

Further, a parameter of individually calibrated Weekly Training Load (WTL) can be used. High WTL may increase the need for sleep and low WTL may decrease the need for sleep. There may be a range where the WTL does not change the need for sleep from the default value, for example when the WTL is moderate and between 2.0 - 3.5.

In an exemplary embodiment, such a method can be implemented in an embedded device having limited CPU and memory resources and having a host system. In one exemplary embodiment, the host system may make use of ETE and THA-libraries (which may generally be referred to as "THA"), wherein an ETE library is a real-time heart rate analysis library, and a THA library is a training history analysis library. In an exemplary embodiment, THA-software may be called and executed temporarily to calculate the need for sleep value.

According to an exemplary embodiment, it may be contemplated to reduce the demand of resources - particularly random-access memory (RAM) or dynamic memory in general - by selecting key variables suitably. In such an exemplary embodiment, the amount of resident memory that may be demanded may be very limited, and the system may be configured to store only characteristics of each exercise and each night's sleep.

Still referring to the exemplary method in Fig. 1, the method may continue with an additional optional exemplary step where sleep periods may be measured from a collection of physiological data 118. The sleep periods may be detected using a heart rate sensor, such as by detecting a heart rhythm indicative of sleep. In an alternate embodiment, the sleep periods may be detected using a motion sensor. A sleep score may be analyzed from the detected sleep periods. The sleep score may be a value that represents the length or quality of the user's sleep. A sleep score may be stored for every sleep period 120 (such as every night). In a final sub-step of the sleep need analysis, a processing unit may analyze the age, training load history, body stress history, and sleep scores to determine a sleep need value 122.

An exemplary second portion of the method illustrated by Fig. 1 may include a calculation of a sleep pressure value 104. To calculate a sleep pressure value 104, the method may first receive the sleep need value previously calculated 124. In a next exemplary step, the method may detect sleep and awake periods 126. Sleep and awake periods may be continuously detected using sensors such as heart rate sensors or motions sensors. By continuously detecting and analyzing sleep and awake periods, the method may provide a user with up-to-date and instantaneous updates regarding their sleep need and sleep pressure. Next, body resources may be analyzed from physiological data such as heart rate variability (HRV) data and motion data 128. Body resources is a term characterizing the dynamic between the physiological load put on the body and the amount of time needed for recovery, and a measurement of body resources may be obtained or estimated based on measurements of physiological load and measurements of recovery time. (For example, a quantitative measurement of body resources, may be a percentile score maintained from 0 to 100, which is adjusted upwards when recovery is detected and adjusted downwards based on physiological load or other depleting factors like stress) Recovering and sleeping may increase resources and, alternatively, stress and physical activity may decrease body resources. High levels of body resources illustrate that the user is well recovered and ready to face the daily events. Low levels of body resources, for their part, may indicate that the user may not be in the best physical or mental condition to perform demanding tasks and it may be recommended that they rest. (Different levels of rest, which may have different effects, may be contemplated; it may be contemplated that, while downtime during the day may be important and may offer some level of recovery, sleep offers the greatest opportunities for recovery. As such, recommendations may include recommendations for further restful moments throughout the day or may include recommendations for further sleep.) In a next exemplary step, the time elapsed from the previous night's sleep may be calculated 130. In an exemplary final step, the sleep need value, sleep and awake periods, physiological data, and time since previous sleep may be compiled to calculate a sleep pressure value 132. The method may produce feedback to the user based on the sleep pressure and sleep need values.

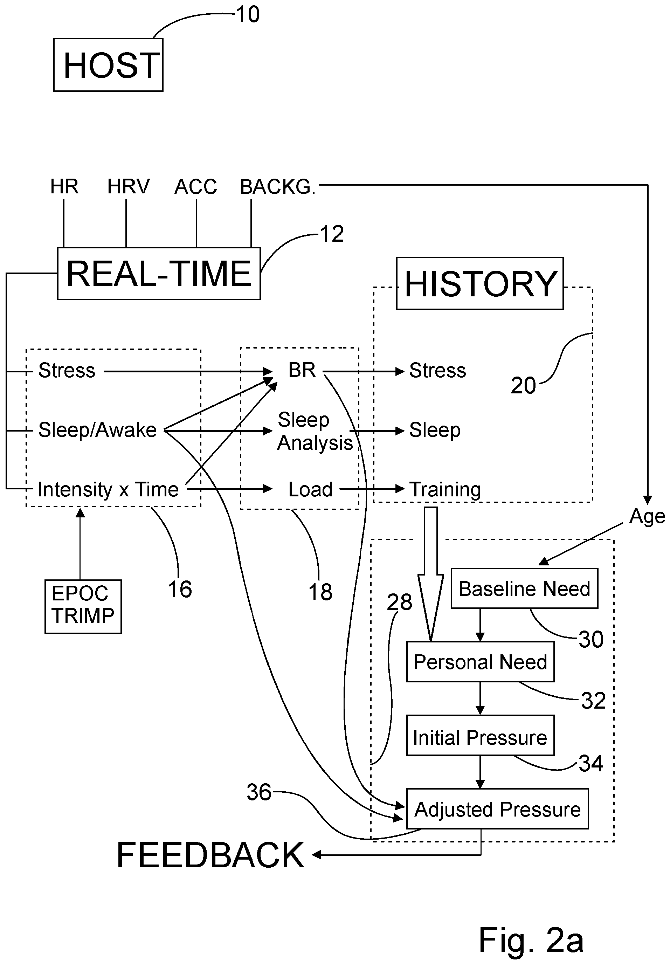

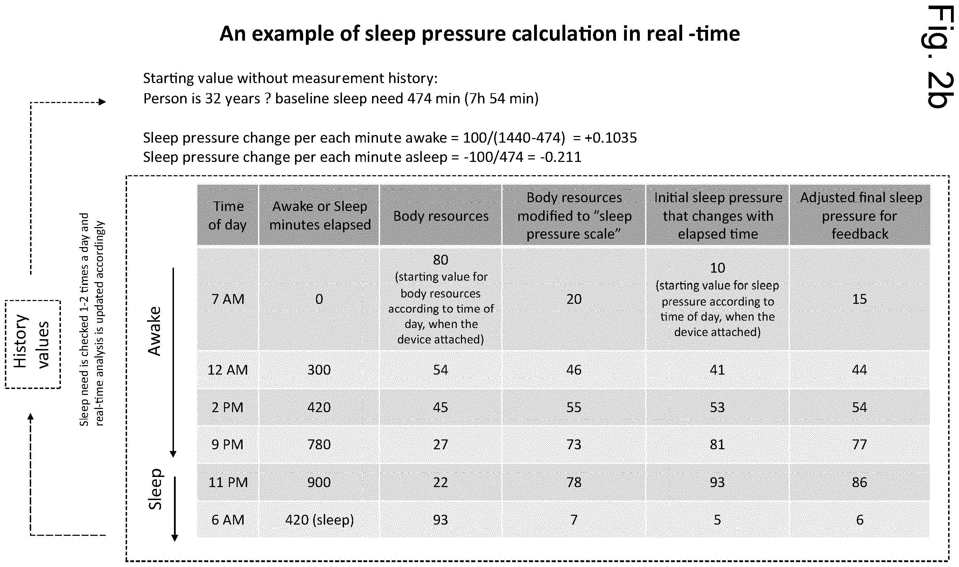

Referring to figures 2a and 2b the operation is depicted more in detail. The HOST-system 10 has real-time ETE library software, which produces results for 24 hour analysis and for timed activities, such as a training session. For timed activities, like a training session, HR, ACC, speed and training time are measured. The ETE-library software has the background information as input (such as age). It estimates EPOC and calculates TRIMP in real-time. The real-time part is run every 5 seconds.

A load value (intensity x time) information is calculated from these. Training parameters WTL and MTL are determined. These values are stored by the host system. (THA) library software picks them when needed. THA -software is called when there is a need to update training load (weekly or monthly). Typically, this occurs once a day or when a new training session is stored.

The host system covers real-time modules 12, 16 and 18. The ETE software also calculates Body resources value in real-time based on stress, recovery and physical activity information. In these calculations HRV, HR, and ACC information (or speed) are needed.

In one embodiment the real-time calculation of sleep pressure is carried by ETE-software, too. The calculation needs information on body resources and real-time detection of Awake/Sleep state. This detection of Awake/Sleep also works by ETE -software.

For the stress, recovery, and sleep score history, there needs to be a similar solution than THA-software. It could be implemented with the THA-software, but one option is to provide a Lifestyle History Library software, block 20, which stores key results or parameters for each day such as average stress-relax intensity (RSI), sleep score and so on. Typically, this Lifestyle History Library (LHT) -software would be called once a day when the ETE is reset for a new day.

The LHT -software would provide information for ETE -software on personal sleep need (similar to current activity class information). Thus, THA -software is used to calculate a person's activity class and ETE -software uses activity class for scaling training information. This personal sleep need would give ETE information on how the real-time information needs to be processed, for example, determine the rate of sleep pressure increase and decrease by each minute (which could also be calculated in 5-sec segments) for that given day and for this person, block 28.

This would mean that the history is not calculated for each 5-sec, rather once a day, and the information on the rate of sleep pressure change would remain the same for that one day. The real-time analysis would be run all the time, but it would not need to be stored. At the end of the day, the sleep pressure value would be stored, and when the ETE is reset for new day that previous day value would be provided to ETE for a starting point to the calculation.

The user can monitor whenever he/she wants the real-time sleep pressure value and it can be shown in some static scale. There are other process, which provide for example feedback sentences. For example, at specific times of the day or when needed, this process calls ETE - software for the current sleep pressure value, and it may call THA -software to check whether there has been, for example, a hard exercise session for that day. This makes it possible to give feedback such as "You still have moderate energy levels, even your vigorous activity increases your recovery needs." There would be also a need to call LHT-software for checking, for example, sleep quality from the previous night to be able to give feedback on how that affects Sleep Pressure for the given day. NOTE: These daily values most probably would still be stored in ETE, and THA and Lifestyle History Library would only be needed for updating the personal sleep need.

As a numerical example, a 32 years old subject has a baseline sleep need of 474 min (7 h 54 min), see the equation on page 9.

The dedicated block 20 counts history data of stress. It is a child process and calls sleep and training and obtains a correction of +25 min.

STRESS:

    Long-term stress level low (RSI = +50), figure 3a
    $\rightarrow$ sleep need -15 min

TRAINING:

    Short-term training load (WTL) = 3, figure 3b
    $\rightarrow$ no effect on sleep need

    Absolute training load in last 7 days = 400 units, figure 3e
    $\rightarrow$ sleep need +10 min

    Long-term training load (MTL) = 4, figure 3c
    $\rightarrow$ sleep need +30 min

SLEEP:

    Sleep score last night = 50, figure 3d
    $\rightarrow$ no effect on sleep need

The person would need -8:15 of sleep (474 min +25 min = 499 min). In this example the expectation for sleep pressure to be compensated with sleep and results in the person to start the morning with low sleep pressure (10 in a 0-100 scale). This may of course be changed by daily activities, stress, etc., that affect body resources. Instant BR and awake/sleep-data have equal weights, when adjusting final pressure value from the initial pressure value, step 36.

An example regarding the stress history and its use in calculation is presented in table 2. Stress indicia (24h Body stress index), once per day, have been stored in memory THA-software calculates a rolling 7-day Average RSI-value when sleep need process is run.

Table 2

| Date | 24h body stress value (Daily RSI average) | Body stress history value (Rolling 7-day Average RSI) | Personal sleep need change to baseline need by stress history |
|---|---|---|---|
| 1.1. | -23 | N.A. | |
| 2.1. | 14 | N.A. | |
| 3.1. | 12 | N.A. | |
| 4.1. | -17 | N.A. | |
| 5.1. | 10 | N.A. | |
| 6.1. | 27 | N.A. | |
| 7.1. | 33 | **8** | **+23 min** |
| 8.1. | 8 | 12 | +19 min |
| 9.1. | -12 | 9 | +22 min |

In the above example the average value is 8, That will cause +23 minutes sleep need change to the baseline value (see figure 3a). An example regarding training load history and its use in calculation is presented in table 3 and 4 below. Training load is (one per an exercise) have been stored in resident memory, THA-software calculates a rolling 7-day Average cumulative value when sleep need process is run. On the date 7.1 there is first time a rolling 7-day cumulative sum (556) available, which gives weekly training load value of "5". All of the memory resident tables together along with the temporal data registry may be relatively small without losing accuracy in sleep need calculation.

Table 3

| Date | Time of day | Training load for training session based on intensity x time (EPOC or TRIMP) | Acute training load | WTL value | Personal sleep need change to Baseline need by WTL |
|---|---|---|---|---|---|
| | | | (rolling 7-day cumulative sum) | | |
| 1.1. | 12:32 | 243 | N.A. | | |
| 3.1. | 11:03 | 22 | N.A. | N.A. | |
| 4.1. | 8:20 | 54 | N.A. | N.A. | |
| 4.1. | 17:34 | 34 | N.A. | N.A. | |
| 5.1. | 11:56 | 176 | N.A. | N.A. | |
| 7.1. | 9:14 | 27 | **556** | **5** | **+60 min** |
| 9.1. | 14:23 | 56 | 369 | 4 | +20 min |
| 12.1. | 8:35 | 32 | 115 | 1 | -20 min |
| 13.1. | 10:10 | 189 | 304 | 3.touko | No change |

More accuracy is achieved when activity class is used relating to exercise analysis.

Table 4 Optional Activity class

| Training load limits for WTL | 0 | 133 | 176 | 219 | 266 | 312 | 396 | **480** |
|---|---|---|---|---|---|---|---|---|
| Corresponding weekly training load (WTL) value | 1,0 | 2,0 | 2,5 | 3,0 | 3,5 | 4,0 | 4,5 | **5,0** |
| Training load limits for WTL are converted to "Weekly training load (WTL) values": Figure 3b gives 60 minutes by value of "5". | | | | | | | | |

Figure 2b shows basic calculation of sleep pressure value combining initial pressure value with instant body resource value and elapsed time and without history data. Body resources are calculated from all three inputs and is scaled in a range of 0 - 100 scaled to "a sleep pressure scale", which is inverse to body resources. Then that inverse body resource and initial sleep pressure according to elapsed time - gives together the adjusted sleep pressure for feedback, in range of 6 - 86.

This value is dependent on history. If there is no history, the personal need is based on age. The personal sleep need modifies the rate of sleep pressure increase/decrease when being awake/sleep. The starting level of the sleep pressure (initial pressure) is related to time of day, for example 10 at 7 AM. Thereafter the initial pressure is adjusted with continuous tracking of sleep/awake and body resources progress. Sleep/Awake analysis counts Sleep/Awake discrete data (0,1,2). Intensity and time (duration) depict the load of an exercise. After each exercise they are stored in resident memory.

Further, it may be noted that high chronic stress (which may also be characterized as low "relax stress intensity," or RSI) increases the need for sleep. Referring generally to Fig. 3A, an average stress level from the last 7 days 112, or some other time period, may be used as a basis for stress analysis 110. Stress level may be measured by the average Relax-Stress Intensity (RSI) parameter where -100 indicates full stress and +100 indicates full relax. There may be limitations of how much data is required for a stress level to be considered in the calculation of sleep need. For example, in one exemplary embodiment illustrated by Figure 3A, at least 24 hours of RSI data may be required including at least 5 hours of sleep data. Now referring to exemplary Figure 3B, high short-term (acute) training load may also increase the need for sleep and low short-term training load may decrease the need for sleep from the default value.

Referring now to Figure 3C, high long-term (chronic) training load may increase the need for sleep and low long-term training load may decrease the need for sleep from the default value. As such, measurement of physiological data 114 may make use of stored data relating to this information 116. A parameter considering individually calibrated Monthly Training Load (MTL) may be used. There may be a range where the MTL does not change the need for sleep from the default value, for example when the MTL is moderate and between 2.0 - 3.0.

Referring now to Figure 3D, poor acute sleep increases the need for sleep. For example, a sleep score parameter from one or more preceding nights on a scale from 0 to 100 may be used to evaluate the restorative effect of previous sleeps.

Referring now to figure 3E, absolutely high training load may increase the need for sleep. As such, measurement of physiological data 114 may make use of stored data relating to this information 116. For example, in a case of professional athletes, their training load may be individually at a moderate level and therefore for example short-term (acute) training load may otherwise not be at the level that would increase the need for sleep. However, compared to ordinary people, an athlete's training load can be very high. This can be taken into account by using an absolute training load function, such as TRIMP_sum or EPOC_sum or another cumulative training load metric.

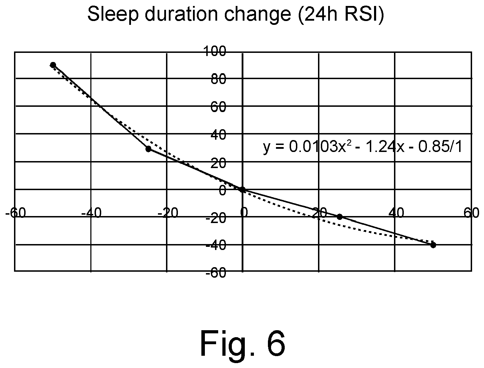

There could be many empiric functions for this purpose. Figure 6 presents another optional function related to relax-stress intensity. However, the further functions does not give such improvement than the above mentioned ones."

In an exemplary embodiment, the sleep need function may be limited to a given interval resolution, such as a 15-minute interval, and also to a predetermined range. This may be based on the user's age, as demonstrated in Table 5:

**Table 5: Exemplary output ranges, by age.**

| Age | Output range |
|---|---|
| 6-13 yrs | Sleep range 9-13 hours |
| 14-17 yrs | Sleep range 8-12 hours |
| 18-64 yrs | Sleep range 7-11 hours |
| 65+ | Sleep range 7-10 hours |

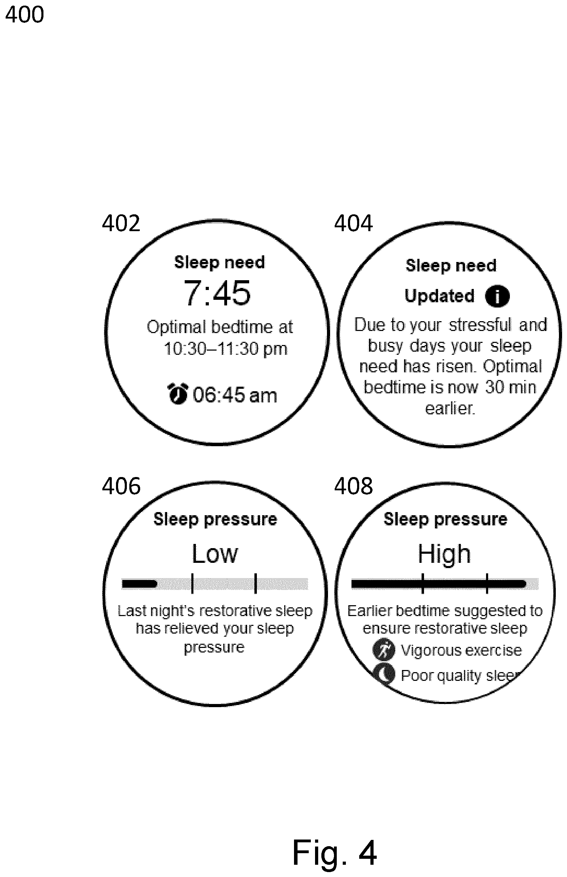

Referring generally to exemplary Fig. 4, the system may be configured to provide individually tailored recommendations for sufficient sleep duration. For example, if a wake alarm has been set or typical awakening time is followed, a recommended bedtime range 402 may be given based on the planned awakening time for the user, though in certain exemplary embodiments it may be contemplated to adjust this recommended bedtime range based on the characteristics of the specific user or based on history data associated with the user (such as the user's usual natural sleep duration in the absence of an alarm or any other applicable lifestyle history data), for the user, though in certain exemplary embodiments it may be contemplated to adjust this recommended bedtime range based on the characteristics of the specific user or based on history data associated with the user (such as the user's usual natural sleep duration in the absence of an alarm or any other applicable lifestyle history data).The recommended bed time can be given, for example within a 60 min slot, where the recommended sleep duration is set in the middle of the slot. The feedback can include various verbal feedback sentences such as those described herein. Further, a sleep need update 404 may indicate an update to the sleep need value, and may be displayed along with the new bedtime and a feedback phrase. A sleep pressure screen may be displayed when the sleep pressure is low 406 or when the sleep pressure is high 408, indicating the level of sleep pressure. Further, the sleep pressure screen may provide feedback or recommendations as well as explanations for the sleep pressure value or level.

For example, the following example feedback sentences may be provided for users with a very low Sleep Need as compared to their age-based default. These may be given when the recommended sleep duration is more than 30 minutes shorter than the age-based estimate. The feedback sentence may be updated when there is new output, i.e. when a recommendation changes to a new 15-minute slot. These are exemplary feedback sentences, and feedback sentences may be structured in a number of ways, not limited to the following. Feedback may also be given when Sleep Need is low, moderate, high or very high compared to an age-based default value. In addition, the feedback sentences can take into account if the sleep need has decreased, remained constant, or increased compared to previous feedback.

**Table 6: Exemplary user feedback, in this case for lessened recommended sleep duration due to one or more factors. (A final case, where all factors are "No," should not be possible, and may indicate an error. This may result in generic feedback such as "Your sleep need is lower than typical.")**

| Stress (RSI) decreases Sleep Need value compared to default | Training load (WTL or MTL) decreases Sleep Need value compared to default | Sleep score decreases Sleep Need value compared to default | Feedback Sentence |
|---|---|---|---|
| Yes | No/N.A. | No/N.A. | Your need for sleep is lower than typical due to your calm and relaxing days. |
| Yes | Yes | No/N.A. | Your need for sleep is lower than typical due to your calm and relaxing days and easy training. |
| Yes | No/N.A. | Yes | Your need for sleep is lower than typical due to your calm and relaxing days and very good sleeping recently. |
| Yes | Yes | Yes | Your need for sleep is lower than typical due to low overall load on your body. |
| No/N.A. | Yes | No/N.A. | Your need for sleep is lower than typical due to your low training load. |
| No/N.A. | No/N.A. | Yes | Your need for sleep is lower than typical due to very good sleeping recently. |
| No/N.A. | Yes | Yes | Your need for sleep is lower than typical due to low training load and very good sleeping recently. |

Table 7: Exemplary user feedback, in this case for slightly reduced recommended sleep duration due to one or more factors. (A final case, where all factors are "No," should not be possible, and may indicate an error. This may result in generic feedback such as "Your sleep need has decreased. Optimal bedtime is now X min later.") For reference, X may be calculated as the difference between previous and current recommended sleep duration. The feedback sentence provided to the user may be updated when there is new output i.e. when a recommended sleep duration changes to new 15 minutes slot.

| Chronic stress decreases recomm. Sleep duration equally or more than previously | Training load (WTL or MTL) decreases recomm. Sleep duration equally or more than previously | Sleep score decreases recomm. Sleep duration equally or more than previously | Feedback Sentence |
|---|---|---|---|
| Yes | No/N.A. | No/N.A. | Your need for sleep has decreased due to your lowered stress levels. Your optimal bedtime is now X min later. |
| Yes | Yes | No/N.A. | Your need for sleep has decreased due to your lowered stress levels and easier training. Optimal bedtime is now X min later. |
| Yes | No/N.A. | Yes | Your need for sleep has decreased due to your lowered stress levels and improved sleeping recently. Optimal bedtime is now X min later. |
| Yes | Yes | Yes | Your need for sleep has decreased due to reduced overall load on your body. Optimal bedtime is now X min later. |
| No/N.A. | Yes | No/N.A. | Your need for sleep has decreased due to your decreased training load. Optimal bedtime is now X min later. |
| No/N.A. | No/N.A. | Yes | Your need for sleep has decreased due to your improved sleeping recently. Optimal bedtime is now X min later. |
| No/N.A. | Yes | Yes | Your need for sleep has decreased due to your decreased training load and improved sleeping recently. Optimal bedtime is now X min later. |

**Table 8: Exemplary user feedback, in this case for slightly increased recommended sleep duration due to one or more factors. (A final case, where all factors are "No," should not be possible, and may indicate an error. This may result in generic feedback such as "Your need for sleep has risen. Optimal bedtime is now X min earlier.")**

| Chronic stress increases recomm. Sleep duration equally or more than previously | Training load (WTL or MTL) increases equally or more than previously | Sleep score increases equally or more than previously | Feedback Sentence |
|---|---|---|---|
| Yes | No/N.A. | No/N.A. | Your need for sleep has risen due your increased stress levels. Optimal bedtime is now X min earlier. |
| Yes | Yes | No/N.A. | Your need for sleep has risen due to your increased stress levels and more demanding training. Optimal bedtime is now X min earlier. |
| Yes | No/N.A. | Yes | Your need for sleep has risen due to your increased stress levels and suboptimal sleeping recently. Optimal bedtime is now X min earlier. |
| Yes | Yes | Yes | Your need for sleep has risen due to increased overall load on your body. Optimal bedtime is now X min earlier. |
| No/N.A. | Yes | No/N.A. | Your need for sleep has risen due to your increased training load. Optimal bedtime is now X min earlier. |
| No/N.A. | No/N.A. | Yes | Your need for sleep has risen due to your insufficient sleeping recently. Optimal bedtime is now X min earlier. |
| No/N.A. | Yes | Yes | Your need for sleep has risen due to your increased training load and insufficient sleeping recently. Optimal bedtime is now X min earlier. |

**Table 9: Exemplary user feedback, in this case for dramatically increased recommended sleep duration due to one or more factors. (A final case, where all factors are "No," should not be possible, and may indicate an error. This may result in generic feedback such as "Your need for sleep is extended.")**

| Chronic stress increases recomm. Sleep duration compared to default | Training load (WTL or MTL) increases recomm. Sleep duration compared to default | Sleep score increases recomm. Sleep duration compared to default | Feedback Sentence |
|---|---|---|---|
| Yes | No/N.A. | No/N.A. | Your need for sleep is extended due to your stressful and busy days. |

(continued)

| Chronic stress increases recomm. Sleep duration compared to default | Training load (WTL or MTL) increases recomm. Sleep duration compared to default | Sleep score increases recomm. Sleep duration compared to default | Feedback Sentence |
|---|---|---|---|
| Yes | Yes | No/N.A. | Your need for sleep is extended due to stressful days combined with demanding training. |
| Yes | No/N.A. | Yes | Your need for sleep is extended due to stressful days and insufficient sleeping recently. |
| Yes | Yes | Yes | Your need for sleep is extended due to high overall load on your body. |
| No/N.A. | Yes | No/N.A. | Your need for sleep is extended due to your demanding training. |
| No/N.A. | No/N.A. | Yes | Your need for sleep is extended due to your insufficient sleeping recently. |
| No/N.A. | Yes | Yes | Your need for sleep has risen due to your increased training load and insufficient sleeping recently. |

As discussed above, a sleep score may also be based on a measure of sleep quality, which may adjust a sleep score based on the measured quality of the sleep. The amount of sleep that a person receives plays a strong role in determining their overall health, but it may also be understood that not all sleep is equal, and sleep quality relates more strongly to overall health than sleep duration. The quality of sleep may be measured for users based on a variety of factors. One such factor is how long it generally takes a person to fall asleep. Generally, if it takes more than 30 minutes to fall asleep, the sleep is of a lower quality than if sleep is attained within 30 minutes. Likewise, sleeping fitfully is an indication of a lower quality of sleep, and waking often throughout the night (such as due to drinking alcohol before bed, having caffeine too late in the day, or some other such contributing factor) disrupts the sleep cycle, further lowering sleep quality. The number of minutes that the sleeper remains awake after waking in the night is also an important factor in determining sleep quality.

Some level of restlessness can be expected for most sleepers, depending on what stage of sleep they spend their time in. For example, in an exemplary embodiment, it may be contemplated to divide sleep into four different stages, which may be identified based on the person's detected activity while sleeping. A general sleep quality calculation may be based on a weighted calculation involving the individual's RSI (relax stress intensity), the user's detected activity in each sleep stage, and the user's detected restlessness. In an exemplary embodiment, sleep stage points may be calculated by equally weighing scores on a scale from 0 to 100 from different sleep stage proportions (or otherwise weighing these values).

**Table 10: Exemplary sleep weights for an adult.**

| Points | 0 | 30 | 60 | 100 | 100 | 60 | 30 | 0 |
|---|---|---|---|---|---|---|---|---|
| Light | 100% | 80% | 70% | 60% | 0% | | | |
| REM | 0% | 10% | 15% | 21% | 30% | 40% | 50% | 60% or more |
| Deep | 0% | 8% | 12% | 16% | >20% | | | |
| Awake | 20% | 15% | 10% | <5% | | | | |

Referring to the above Table 10, it may be contemplated that an individual, in this case an adult, may have periods of the night in which they are in a light sleep, in REM sleep, in deep sleep, and briefly awake. It may be recommended that a particular adult individual have around 21% to 30% REM sleep every night, upwards of 16% deep sleep every night, and less than 5% awake time every night, with the remainder being light sleep. Deviations from this pattern may have effects such as are contemplated in the above table, with the person receiving different scores depending on how much of their sleep was in each stage. So, a person that is in a light sleep stage 70% of the time, a REM sleep stage 10% of the time, a deep sleep stage 10% of the time, and an awake state 10% of the time may receive, according to the above chart, 60 points based on their light sleep activity, 30 points based on their REM sleep activity, 30 points based on their deep sleep activity (while in this case they slept more than the 8% value provided for 30 points, they did not achieve the 12% threshold for 60 points, though it may also be contemplated to provide a sliding scale in which they would receive 45 points or some other intermediate value, if desired) and 60 points based on the amount of time that they were awake. Averaging these four values leaves the user with a score of 45, indicating that their sleep was generally at a moderate level. Generally, it may be provided that reaching the recommended age-based sleep duration would result in 100 points, with the poor limit (30 points) being 70% of the recommended sleep duration, and the low limit (0 points) being 25% of the recommended sleep duration.

It may be contemplated that certain attributes of the individual, such as age, gender, and so forth, may be incorporated into this analysis. For example, while adults may optimally receive around 21-30% REM sleep, with excess REM sleep generally not indicating good sleep quality, newborns may spend about 40% or more in REM to achieve a good sleep quality.

It may also be contemplated that, in some circumstances, it may not be possible to determine a sleep state of the user. For example, it may not be possible to readily distinguish between a light sleep state and a resting-awake state for a particular individual based on the collected data (such as motion or heartbeat data) or between certain other sleep states. Instrumentation, too, may not be perfect, and it may be contemplated that a device may lose signal or lose power at some point during the night. For example, a user may roll over onto, and inadvertently hold down, a power button, preventing data from being collected at all. In such circumstances, it may be contemplated to score the individual based on what data can be collected. For example, if certain data is incorrect or missing, yet certain data is available past a certain threshold (for example, two continuous hours of data), this data may be extrapolated to be generally reflective of the user's sleep state. If certain data cannot be properly read, then data that can be read (such as the user's REM sleep) may be used as the basis for scoring, with only this data being used as part of the average.

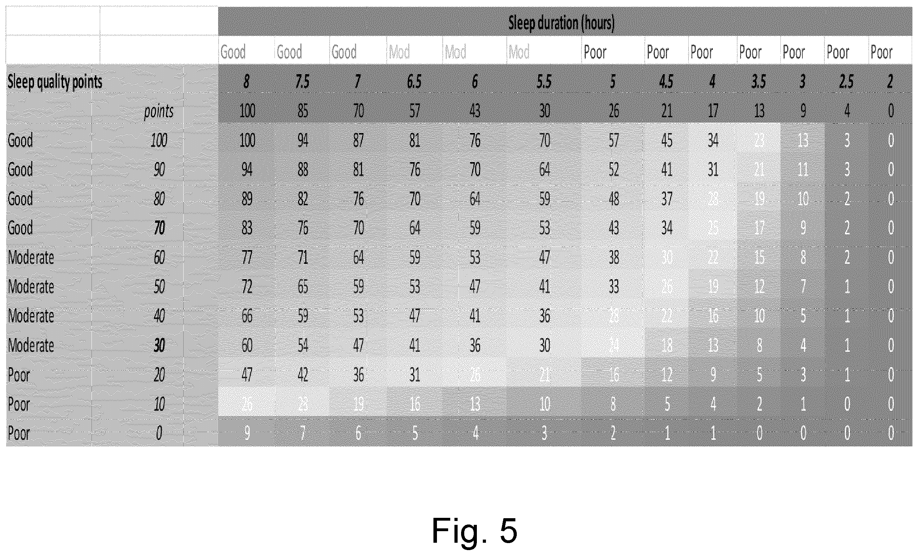

As noted above, it may be contemplated that a user who has slept according to the 70/10/10/10 pattern discussed above may have approximately 45 sleep quality points. Referring to Figure 5, this value may be used in order to determine an adjusted sleep score based on a predetermined relation.. For example, in a first instance, an adjusted sleep score may be based on the relation $((0.57*SQ)+(0.43*SD))*MIN(1;(SD-3)/27)*MIN(1;(SQ+6)/30)$, with SQ representing a sleep quality score and SD representing a sleep duration score. In such a relation, it may be contemplated that a user that sleeps 8 hours with the aforementioned 70/10/10/10 breakdown, scoring 45 sleep quality points, may have an adjusted sleep score of $25.65+43*MIN(1.97127)*MIN(1.51/30) = 69$ points. These calculations can be seen in the table of Figure 5. In Figure 5, calculations for 40 and 50 sleep quality points at this stage are shown, as adjusted sleep scores of 66 and 72 respectively. It may of course be contemplated to make use of an alternate formula for calculating a final adjusted sleep score, such as the relation Final sleep score = ((2/3 x sleep quality score) + (1/3 x sleep duration score)) x MIN [1, (sleep quality score+10)/40] x MIN[1, (sleep duration score+1)/40].

As noted, the quality of an individual's sleep may also be determined based on the individual's "restlessness" score. Restlessness quantifies how many sudden movements or "arousals" occur during sleep. These sudden movements may correlate with shifts to the lightest sleep stage S1, even though some shifts to S1 are not visible in the accelerometer data. The arousals may be counted using the same activity measure that is used for sleep stage classification (five-second sum of the 1-norm of the difference of the acceleration vector), and using the same threshold value (constant SLEEP_DETECT_ACT_THRESHOLD) that separates stillness from movement. To avoid counting a single continuous period of movement as many arousals, a cooldown period may be enforced. After an arousal, a period of 30 seconds of complete stillness (activity below SLEEP_DETECT_ACT_THRESHOLD/4) may be required before a new arousal is detected. The total number of arousals during the sleep period may be normalized by dividing with a nominal sleep duration of 8 hours and converted to a score from 0 to 100 using a formula. It may be contemplated to calculate restlessness points based on a determination of the percentage of short 1 minute or shorter immobile periods compared to the overall number of immobile periods plus the percentage of all mobile periods between sleep start and end. That is, if the user is frequently moving, and has a high number of very short immobile periods broken up by movement, then the user's sleep may be more restless. The overall result may be then scaled to database percentile results. Essentially, the system may function in this case to obtain movement data-based indicators of fragmented sleep. Movement data may be obtained using a smartwatch or smartphone's accelerometer and/or gyroscope sensors.

The calculations may also make use of a typical bed time score (sleep start time), which may be an average sleep start time from the last 7-day window, and a typical awake time score (sleep end time), which may be an average sleep end time from the last 7-day window.

Just as it may be contemplated to provide a user with feedback based on an unadjusted sleep score (or set of sleep scores), it may be contemplated to provide a user with feedback based on an adjusted sleep score or set of sleep scores. For example, the below table provides a set of potential phrases that may be provided under particular circumstances.

**Table 11: Exemplary single night feedback phrases.**

| General phrase number | Sleep duration | Sleep quality | Data requirements | General feedback phrases (ENG) | Grouping | Label |
|---|---|---|---|---|---|---|
| 1# | Good | Good | Both sleep scores given | You slept long enough and your sleep quality was good. | Good structure | Label 13 |
| 2# | Good | Moderate | Both sleep scores given | You slept long enough and your sleep quality was moderate | Good structure | Label 13 |
| 3# | Good | Poor | Both sleep scores given | You slept long enough but your sleep quality was poor, which may weaken your alertness. | Poor structure | Label 7 |
| 4# | Moderate | Good | Both sleep scores given | Your sleep was of good quality although shorter than recommended. | Good structure | Label 13 |
| 5# | Moderate | Moderate | Both sleep scores given | Your sleep was of moderate quality and shorter than recommended. | - | |
| 6# | Moderate | Poor | Both sleep scores given | Your sleep was of poor quality and shorter than recommended. Your performance level may compromised today. | Poor structure | Label 7 |
| 7# | Poor | Good | Both sleep scores given | Your sleep was too short but it was of good quality. | Short sleep | Label 15 |
| 8# | Poor | Moderate | Both sleep scores given | Your sleep was too short and it was of moderate quality. Your performance level may be compromised today. | Short sleep | Label 15 |
| 9# | Poor | Poor | Both sleep scores given | Your sleep was too short and of poor quality. Take it easy today. | Short sleep, Poor structure | Label 7 & 15 |

Detailed information may also be given, based on patterns or other detected information, such as alcohol use or exercise. For example, an exemplary set of detailed information that may be provided based on alcohol use, exercise, stress, high altitude, and other factors may be provided in the below table. As previously stated, it may also be contemplated to have combinations of such factors result in different feedback.

**Table 12: Exemplary single night feedback phrases.**

| Detailed phrase number | When overall score is below 60 plus there is: | Definition | Data requirements | Detailed feedback phrases (ENG) | Grouping | Label |
|---|---|---|---|---|---|---|
| 1# | Low alcohol use + exercise | Alcohol doses 1-3 men or 1-2 women and TE 3.5 or higher per day. | At least 5h data from when the user is awake, and information on alcohol and both sleep scores given. | Strenuous exercise and even small amounts of alcohol together may weaken recovery during sleep. | Alcohol, Exercise-load | Label 1 & Label 3 |
| 2# | A lot of alcohol + exercise | Alcohol doses 4 or more in men or 3 or more in women, and daily at 3.5 or higher. | At least 5h data from when the user is awake, and information on alcohol and both sleep scores given. | Strenuous exercise and alcohol together may weaken recovery during sleep. | Alcohol, Exercise-load | Label 1 & Label 3 |
| 3# | A little alcohol use + a very stressful day | Alcohol doses from 1 to 3 men or 1-2 women and Average RSI during a period where the user is awake -100 - (-50). | At least 5h data from when the user is awake, and information on alcohol and both sleep scores given. | Very high stress level and even small amounts of alcohol together may weaken recovery during sleep. | Alcohol, High stress | Label 1 & Label 4 |
| 4# | A lot of alcohol + a very stressful day | Alcohol doses 4 or more in men or 3 or more in women, and Average RSI during a period where the user is awake -100 - (-50). | At least 5h data from when the user is awake, and information on alcohol and both sleep scores given. | Very high stress levels and alcohol together may weaken recovery during sleep. | Alcohol, High stress | Label 1 & Label 4 |
| 5# | A little alcohol + late to sleep | Alcohol doses 1-3 men or 1-2 women and sleep between 01-08. | At least 5h data from when the user is awake, and information on alcohol and both sleep scores given. | Even small amounts of alcohol together with a late bed time negatively impacts sleep. | Alcohol, Poor sleep rhythm | Label 1& Label 5 |

(continued)

| Detailed phrase number | When overall score is below 60 plus there is: | Definition | Data requirements | Detailed feedback phrases (ENG) | Grouping | Label |
|---|---|---|---|---|---|---|
| 6# | A lot of alcohol + late to sleep | Alcohol doses 4 or more in a man or 3 or more women, and sleep between 01-08. | At least 5h data from when the user is awake, and information on alcohol and both sleep scores given. | Alcohol together with a late bed time negatively impacts sleep. | Alcohol, Poor sleep rhythm | Label 1 & Label 5 |
| 7# | A little alcohol | Alcohol doses 1-3 men or 1-2 women. | Information on alcohol and both sleep scores given. | Even small amounts of alcohol in the evening may weaken recovery during sleep. | Alcohol | Label 1 |
| 8# | Moderate alcohol | Alcohol doses 4-6 men or 3-5 women. | Information on alcohol and both sleep scores given. | Alcohol may significantly worsen recovery during sleep. | Alcohol | Label 1 |
| 9# | A lot of alcohol | Alcohol doses> 6 in men or> = 6 in women. | Information on alcohol and both sleep scores given. | High dosage of alcohol significantly weakens recovery during sleep. | Alcohol | Label 1 |
| 10# | High standing + acclimatization | Acclimatization in progress (acute adaptation phase) | Information on altitude and acclimatization, and both sleep scores given. | High altitude may have negative effects on sleep quality. | Altitude | Label 2 |
| 11# | Extremely heavy exercise | TE5 workout a day. | At least 5h data from when the user is awake and both sleep scores given. | Very strenuous exercise might disturb sleep. | Exercise-Load | Label 3 |
| 12# | Loads of exercise within 10h | TE4 within 10 hours before sleep. | At least 5h data from when the user is awake and both sleep scores given. | Strenuous exercise together with other stress factors can weaken recovery during sleep. | Exercise-Load | Label 3 |
| 13# | Loaded exercise within 3-5h | TE4-4.9, 5h before sleep period OR 3-3.9, 3h before bedtime. | At least 5h data from when the user is awake and both sleep scores given. | Strenuous exercise can weaken recovery during sleep. | Exercise-Load | Label 3 |

(continued)

| Detailed phrase number | When overall score is below 60 plus there is: | Definition | Data requirements | Detailed feedback phrases (ENG) | Grouping | Label |
|---|---|---|---|---|---|---|
| 14# | Exercise near bedtime | TE2.5-3, 2h before sleep period OR TE1.5-2.5, with a duration of at least 90min, within 2h before the sleep period. | At least 5h data from when the user is awake and both sleep scores given. | Exercising too close to bed time can negatively affect sleep. | Exercise-Load | Label 3 |
| 15# | A very stressful day | Average RSI during a period where the user is awake -100 - (-50). | At least 5h data from when the user is awake and both sleep scores given. | Your very high stress level during the day can make it difficult to calm down and relax during sleep. Stress is a typical factor disturbing sleep. | High stress | Label 4 |
| 16# | Stressful day | Average RSI during a period where the user is awake -50 - (-30). | At least 5h data from when the user is awake and both sleep scores given. | Your high stress level during the day may weaken your recovery during sleep. | High stress | Label 4 |
| 17# | Jet lag | Time zone shift (new input required) | Both sleep scores given. | Jet lag can affect the body's biological rhythms and acutely negatively impact sleeping. | Poor sleep rhythm | Label 5 |
| 18# | Late to sleep | Sleeping time between 00-08. | Both sleep scores given. | Late bed time does not optimally support sleep. | Delayed sleep rhythm | Label 16 |
| 19# | Variable bedtime | The standard deviation of sleep time over 60 min. | Bed times for at least 2 consecutive nights | Changing bed times from one night to another does not optimally support sleep. | Poor sleep rhythm | Label 5 |
| 20# | Inactive day | FirstbeatPointsPhysicalActivity (or another calculated physical activity score from a fitness tracker) less than 30. | At least 5h data from when the user is awake and both sleep scores given. | Low amount of daily activity can negatively impact recovery during sleep. | Inactivity | Label 6 |
| 21# | Deep sleep a little | Deep sleep points less than 30. | Both sleep scores given. | Sleep was light and lacking the most restorative deep sleep phases. | Poor structure | Label 7 |

(continued)

| Detailed phrase number | When overall score is below 60 plus there is: | Definition | Data requirements | Detailed feedback phrases (ENG) | Grouping | Label |
|---|---|---|---|---|---|---|
| 22# | Restlessness from the early days | Restlessness score high in the early part of night | Both sleep scores given. | Sleep was restless in the beginning. | Poor structure | Label 7 |
| 23# | Restlessness from late night | Restlessness score high in the early part of night | Both sleep scores given. | Sleep was restless towards the end. | Poor structure | Label 7 |
| 24# | Work cycle before bedtime. | A work cycle is indicated in the 1h15min window before the start of the sleep period. | At least 5h data from when the user is awake and both sleep scores given. | Working just before going to bed can negatively impact sleep. | Work | Label 8 |
| 25# | Weak condition | Fitness classification very weak or weak | Fitness classification + both sleep scores given. | Low level of fitness may weaken the ability to recover. | Poor fitness | Label 9 |
| 26# | Frequent restless motion during the night | Awake score = 0 | Both sleep scores given. | There was a lot of time spent awake during the sleep period. | Poor structure | Label 7 |
| - | - | - | - | - | - | - |
| 30# | A little alcohol | Alcohol doses 1-3 men or 1-2 women. | At least 5h data from awake, and information on alcohol and both sleep scores given. | Even small amounts of alcohol may weaken recovery, however, sleep was still good. | Alcohol | Label 1 |
| 31# | A lot of alcohol | Alcohol doses 4 or more in men or 3 or more women. | At least 5h data from awake, and information on alcohol and both sleep scores given. | Alcohol weakens recovery, however, sleep was still good. | Alcohol | Label 1 |
| 32# | High stay + acclimatization | Acclimatization in progress (acute adaptation phase) | Information on altitude and acclimatization, and both sleep scores given. | You slept well although your body has not yet fully acclimatized to high altitude. | Altitude | Label 2 |
| 33# | A very stressful day | Average RSI during a period where the user is awake -100 - (-50). | At least 5h data from awake and both sleep scores given. | You slept well despite a very high stress level during the day. | High stress | Label 4 |

(continued)

| Detailed phrase number | When overall score is below 60 plus there is: | Definition | Data requirements | Detailed feedback phrases (ENG) | Grouping | Label |
|---|---|---|---|---|---|---|
| 34# | Stressful day | Average RSI during a period where the user is awake -50 - (-30). | At least 5h data from awake and both sleep scores given. | You slept well despite a high stress level during the day. | High stress | Label 4 |
| 35# | Low stress level per day. | Average RSI during a period where the user is awake less than - 30. | At least 5h data from awake and both sleep scores given. | Low stress level during the day supports recovery during sleep. | Low stress | Label 10 |
| 36# | Recurring moments were included in the day. | FirstbeatPointsStressBalanceRecoveryAwake (or another stress calculation score) 60 or higher. | At least 5h data from awake and both sleep scores given. | Relaxing moments during the day support recovery during sleep. | Low stress | Label 10 |
| 37# | Returning moments before going to bed. | At least 30 mins recovery state during 2h period before sleep start. | At least 5h data from awake and both sleep scores given. | Relaxing moments before going to bed support recovery during sleep. | Low stress | Label 10 |
| 38# | Suitable exercise / active day | FirstbeatPointsPhysicalActivity 60 or higher. | At least 5h data from awake and both sleep scores given. | Being physically active during the day enhances recovery during sleep. | Good activity | Label 11 |
| 39# | The start of recovery was delayed | Less than 5 min recovery in the first hour of sleep. | Both sleep scores given. | Recovery during sleep was good, but the start of recovery was delayed. | High stress | Label 4 |
| 40# | Suitable bedtime and regular sleep rhythm (long-term follow-up, not one night) | Sleeping time between 21-24, and bedtime standard deviation less than 25 min. | Bed times for at least 2 consecutive nights | Your regular bed times support your recovery during sleep. | Good sleep rhythm | Label 12 |
| 41# | Late to sleep | Sleeping time between 00-08. | Both sleep scores given. | You slept well although went to bed late, which typically impairs sleep. | Delayed sleep rhythm | Label 16 |
| 42# | Good sleep structure | Sleep stage overall score over 80. | Both sleep scores given. | Sleep was deep and calm, which optimally supports long-term wellbeing. | Good structure | Label 13 |
| 43# | In good condition | Fitness classification is good-superior | Fitness classification + both sleep scores given. | Your high level of fitness enhances recovery during sleep. | Good activity | Label 14 |

It may also be contemplated to provide feedback to a user based on their results over a period of time. In an exemplary embodiment, such feedback may be triggered when a user has received the same sleep feedback for a certain number or large proportion of days. The most frequently given label may determine the feedback sentence. If no label rules apply, or no label rules apply to the whole of this period, then an average sleep score may be calculated from the available data and used as the basis for feedback over this time period. The following table describes possible feedback phrases that may be presented when the same feedback is given multiple times in a given time period.

Table 13: Exemplary multiple night feedback phrases.

| Label-based phrase number | When these functions are regularly repeated | Definition | Data requirements | Label feedback phrases (ENG) |
|---|---|---|---|---|
| 60# | Alcohol | Label 1 | At least 3 sleep feedbacks given during the window. | Alcohol seems to have weakened your ability to recover during sleep. |
| 61# | Altitude | Label 2 | as above | High altitude seems to affect your ability to recover during sleep, especially before your body has adapted to the conditions. |
| 62# | Altitude + still good sleep | Label 2 + average sleep score over 60 | as above | You are sleeping well although your body has not yet fully acclimatized to high altitude. |
| 63# | Exercise-Load | Label 3 | as above | Load caused by exercising seems to negatively impact your sleep. Pay attention to the balance between training and rest, as well as the timing of training sessions. |
| 64# | High stress + still good sleep | Label 4 + average sleep score over 60 | as above | You seem to be under high stress, but you are still sleeping well. It is good to pay attention to stress and try to lower stress level in order to recover well in the future too. |
| 65# | High stress | Label 4 | as above | You seem to be under high stress. Focus on finding relaxing moments, healthy nutrition and easy physical activity to support your sleep. |
| 66# | Poor sleep rhythm | Label 5 | as above | You seem to struggle with changing sleep rhythms. Try focusing on keeping the same bed and awakening times, and support this with regular physical activity. |
| 67# | Inactivity | Label 6 | as above | Getting more physical activity could enhance your sleep and be beneficial for fitness. |
| 68# | Poor structure | Label 7 | as above | Your sleep quality might need improvement. Try finding ways to ensure a higher quality of sleeping. |
| 69# | Work | Label 8 | as above | Working close to bed time can have a negative impact on sleep. It may be beneficial to focus on this behavior. |

(continued)

| Label-based phrase number | When these functions are regularly repeated | Definition | Data requirements | Label feedback phrases (ENG) |
|---|---|---|---|---|
| 70# | Poor fitness | Label 9 | as above | Improving fitness could be one way to enhance the ability to recover and sleep quality. |
| 71# | Short sleep | Label 15 | as above | You sleep less than recommended. Longer sleep would support your long-term wellbeing and energy levels. |
| 72# | Delayed sleep rhythm | Label 16 | as above | Your bed time is typically late. Earlier bed times could enhance the restorative effect of sleep. |
| - | - | - | - | - |
| 80# | Good structure | Label 13 | as above | Your night's sleeps are restful and recovering which supports your long-term wellbeing and energy levels. Well done! |
| 81# | Low stress | Label 10 | as above | Your stress-recovery balance is good, and your recovery during sleep seems to be just fine. Great! |
| 82# | Good activity | Label 11 | as above | Your level of physical activity supports your sleep. Good work! |
| 83# | Good sleep rhythm | Label 12 | as above | You seem to master keeping a regular sleep rhythm, which has positive effects on your body's biorhythms and supports sleeping. Good work! |
| - | - | - | - | - |
| 90# | | sleep points AVER. more than 80 | as above | You sleep enough and your recovery during sleep is excellent. Great! |
| 91# | | sleep points AVER. 60-80 | as above | You seem to sleep enough and recover well. Great! |
| 92# | | sleep points AVER. 30-59 and sleep points ≥60 over 60% of the measuremen t days | as above | Your recovery during sleep is generally good. |
| 93# | | sleep points AVER. 30-59 and sleep points ≥60 at least 33% of the measuremen t days | as above | Your recovery during sleep is moderately good. Pay attention to factors which may negatively affect your sleep. |
| 94# | | sleep points AVER. 30-59 | as above | Your recovery during sleep is moderate. Pay attention to factors which may negatively affect your sleep and seek ways to improve your sleep. |
| 95# | | sleep points AVER. <30 | as above | Your recovery during sleep is not sufficient. It is important to detect sleep disturbing factors and make concrete plans to support sleep. |

When the individual's sleep need is calculated, such as in step 102, a second step 104 may involve the calculation of momentary sleep pressure. This calculation may use the sleep need as an input 124. If the sleep need is not individually known, a default sleep need value can be used based on background parameters, such as age and gender. For example, with 18 to 64-year old adults the default sleep need value may be 8 hours.

In an exemplary embodiment, the sleep pressure may be a function with an output value which is a positive integer. In an exemplary embodiment, 0 indicates the lowest sleep pressure and the sleep pressure may increase as the need for sleep increases. A specific sleep pressure value may indicate an optimal time to go to sleep. Alternatively, if a user's sleep pressure value is too high it may reach what is known as the "danger zone", wherein functions may be impaired due to the lack of sleep. For example, a sleep pressure value of 100 may indicate the optimal time for a user to go to sleep, and the value may continue to increase, indicating that the optimal time to go to sleep has passed and the need for sleep is increasing.

In an exemplary embodiment, sleep pressure may be calculated based on the following analysis. The sleep need, as calculated in step 102, may be an input 124 to the sleep pressure calculation. Further, information regarding how long the user is awake and asleep may be considered in step 126. The user's momentary body status can be considered by evaluating the accumulation of body load from the given day in the body resources step 128. Body status is based on recognition of the current state a person is in, such as "exercise" (and the exercise's respective intensity level), "recovery from exercise", "recovery", "stress", "device off', or "unrecognized". These states provide further definitions of how quickly the body load is increasing or decreasing. Body load is accumulated in particular when a person is in a state that would decrease body resources, such as "exercise" and "stress". Thus, a high body load may indicate that the person's body resources are rapidly depleting. In an exemplary embodiment, the body load of step 128 may be calculated using HRV and acceleration (motion) data. The time elapsed from the previous night's sleep period 130 may also have an impact on the sleep pressure analysis. Finally, the above factors are calculated using a sleep pressure function and feedback is provided to the user 132.

By using the above factors, the sleep pressure value may be calculated with the following steps. First, the pace of sleep pressure increase may be calculated for each time interval spent awake. This can be done by subtracting the sleep need from the amount of time in a full day. Therefore, the increase of sleep pressure in each minute when the user is awake may be calculated with the following function: F1 =100 / (1440 - recommended sleep duration in minutes). For example, if the sleep need is 9 hours, then the change of Sleep Pressure for each hour spent awake would be 100 / (1440 [min] - 540 [min]) = 0.11 units per minute (OR 6.7 units per hour) when user is in the AWAKE state.

Likewise, sleep pressure may decrease for each time interval spent sleeping. This can be calculated with a function. For example, the following function may calculate a decrease in sleep need: F2 = 100 / recommended sleep duration. Therefore, sleep pressure may decrease constantly (100 / recommended sleep duration) when sleeping. To show an example case of how this may be contemplated (using a simplified linear interpolation process) with a person having sleep need of 9 hours (540 minutes), the sleep pressure may decrease by 100 / 540 = 0.185 units per minute (OR 11.1 units per hour) when the user is in the SLEEP state. It may of course be contemplated, in some exemplary embodiments, to have the decrease in sleep pressure be modeled by a non-linear function rather than by linear interpolation; for example, it may be contemplated to provide a logarithmic or power function whereby a sleep pressure will decrease more quickly at the very beginning of the sleep period and then gradually taper off, slowing the rate of decrease after a certain number of hours have passed by. It may likewise be contemplated to make use of multiple functions and to select an appropriate function for a particular sleeper, based on, for example, demographic information about the sleeper or other such information as is desired.

It may also be contemplated to take time of day into account as a factor when calculating sleep pressure, as a factor independent of the user's wake time and sleep time. For example, users may be under greater sleep pressure when it is dark outside, and under lesser sleep pressure when it is light outside. In an exemplary embodiment, a different set of feedback messages may be displayed to the user when the user has a certain sleep pressure score in the morning, in the afternoon, and in the evening, as follows:

**Table 14: Exemplary feedback phrases for sleep pressure in the morning. This may be calculated based on, for example, the relation "Time from awake OR typical awake time < (24h-recommended sleep duration)/3."**

| Sleep pressure value | Other rules | Feedback Sentence |
|---|---|---|
| 0-33 | N.A. | You have high energy levels to focus on your daily activities. |
| 0-33 | Good sleep score | You have high energy levels to focus on your daily activities due to your restful sleep. |
| 34-60 | N.A. | You have moderate energy levels to focus on your daily activities. |

(continued)

| Sleep pressure value | Other rules | Feedback Sentence |
|---|---|---|
| 34-60 | Vigorous exercise (TE over 3) | You have moderate energy levels for your daily activities after vigorous activity. |
| 34-60 | Poor sleep score | Your sleep last night did not optimally relieve your need for sleep. |
| 34-60 | Vigorous exercise (TE over 3) AND poor sleep score | You have moderate energy levels for your daily activities due to poor sleep and vigorous activity. |
| 61-80 | N.A. | You have lowered energy levels for today's activities. |
| 61-80 | Poor sleep score | Your sleep last night was poor. Take it easy today. |
| 61-80 | Vigorous exercise (TE over 3) | There is a quite large load on your body after vigorous activity. Take the rest of the day easy. |
| 61-80 | Vigorous exercise (TE over 3) AND poor sleep score | Your overall load is high, and your body may need some rest. Take the rest of the day easy. |
| 81-100 | N.A. | Your overall load is high, and you seem to suffer some sleep debt. Your body may need more rest. |

**Table 15: Exemplary feedback phrases for sleep pressure in the afternoon. This may be calculated based on, for example, the relation "Time from awake OR typical awake time > (24h-recommended sleep duration) /3 but > ((24h-recommended sleep duration)/3))*2)."**

| Sleep pressure value | Other rules | Sentence |
|---|---|---|
| 0-40 | N.A. | You still have lots of energy for activities. |
| 0-40 | Good sleep score | You still have lots of energy for activities enabled by your restorative sleep last night. |
| 41-60 | N.A. | There is some need for sleep, but not too much yet! |
| 41-60 | Vigorous exercise (TE over 3) | You still have moderate energy levels, but your vigorous activity increases your recovery needs. |
| 61-75 | N.A. | The sleep pressure is piling up due to the demands of the day. |
| 76-100 | N.A. | An earlier bedtime is suggested to ensure restorative sleep. Take the rest of the day easy. |
| 76-100 | Vigorous exercise (TE over 3) | Your need for restorative sleep is increased due to vigorous training. Take the rest of day easy. |

**Table 16: Exemplary feedback phrases for sleep pressure in the evening. This may be calculated based on, for example, the relation "Time from awake OR typical awake time > ((24h-recommended sleep duration)/3) *2)."**

| Sleep pressure value | Other rules | Sentence |
|---|---|---|
| 0-50 | N.A. | You have had a calm day, reducing your need to sleep. |
| 51-70 | N.A. | It's been an energetic day today. |
| 71-84 | N.A. | You have had good energy levels today, but your body is awaiting some quality sleep soon. |
| 71-84 | Vigorous exercise (TE over 3) | Your need for restorative sleep is increased due to vigorous training. Make sure to go to bed early enough. |
| 85-95 | N.A. | Your day has been demanding. Make sure to go to bed early enough to ensure restorative sleep. |
| 85-95 | Vigorous exercise (TE over 3) | You have spent a lot of energy today. Make sure to get some rest soon. |
| 96-100 | N.A. | You may need some rest now. |

Sleep pressure may also be adjusted based on the user's daily lifestyle factors. In a further embodiment, the calculation of sleep pressure may be constantly adjusted based on the accumulation or depletion of the user's body resources, which may be calculated based on daily lifestyle factors such as stress, recovery and physical activity. This physiological data may be obtained by measuring heart rate information, such as heart rate variability (HRV) data, or motion data. In this manner, the Sleep Pressure may increase faster when there is stress or physical activity affecting resource accumulation and slower when there is a recovery state. The body resources-accumulation can be calculated on a scale from 0 to 100 where different physiological states such as stress, recovery and physical activity and their intensity have different slopes. The physiological resources accumulation parameter can also take into account when the data is available and start from a predetermined value (which may be determined, for example, based on the time of day) after a measurement break, resetting the device from when the device is first attached. The effect of physiological status on sleep pressure may be calculated each moment with the following function: F3 = 100 - Body Resources value, where a Body Resource value of 100 would indicate full body resources. For example, a person with full body resources (thus has a Body Resources value of a 100) who has had a lot of physiological recovery may lead to a sleep pressure calculation of 100 - 100 = 0. In the morning, two hours after awakening and with some moderate level of physical activity, this Body Resources value may instead be 70, and sleep pressure may therefore be 30. Alternatively, the opposite implementation may be contemplated, where a higher body resources value may indicate a higher sleep pressure value.

In an exemplary embodiment, the timing of when the analysis process gets HRV data from the user may be accounted for in the calculation of sleep pressure according to functions F1 and F2 equally, rather than when evaluating body resources with F3. For example, if the device is attached for the first time after reset, or for the first time after some specific duration has passed (for example, 6 hours) without data, at 7 A.M., the starting value of sleep pressure may be 10, and at 11 PM the starting value may be 90. Between these time points the starting value of Sleep Pressure may be defined linearly between 10 and 90 according to the time of the day.

The final, momentary Sleep Pressure value may be calculated continuously based on functions F1, F2, and F3. F1 and F2 can be summed constantly, and the resulting function F4 may be averaged with F3. Therefore, a sleep pressure value may be calculated by constantly considering whether the user is asleep or awake and averaging this with physiological body resource parameter. Both functions (F4 and F3) may or may not have equal weight for the final result. For example, an increased weight for the higher value of the functions may be given, with an average of the two functions being calculated based on, for example, an average of w1*F4 and w2*F3, with each of w1 and w2 being weight values. Other calculation methods other than linear averaging may also be used in order to determine a final result; for example, it may be contemplated to use a root-mean-square calculation of F4 and F3 (e.g. $\sqrt{\frac{F4^2+F3^2}{2}}$ ) in order to produce a final result, so as to ensure that the function having the higher value has an increased weight.

For example, for a user who 2 hours after awakening has a Body Resources value of 70, the final sleep pressure value is: 0.5 (2 x 6.7) + 0.5 (100-70) = 6.7 + 15 = 21.7. 21.7 may be considered a low value. In another case, a user 12 hours after awakening has a Body Resources value of 20, the final sleep pressure value may be: 0.5 (12 x 6.7) + 0.5 (100-20) = 40.2 + 40 = 80.2, which may be considered a high value. In a yet another case, for a user 17 hours after awakening with a Body Resources value of 20, the final sleep pressure value may be: 0.5 (17 x 6.7) + 0.5 (100-20) = 56.95 + 40 = 96.95, which may be considered a very high value.

In an exemplary embodiment, feedback for the user can be given with numerical or verbal indicators. For example, according to an exemplary embodiment, the following scaling values can be used.

Table 17: Exemplary scaling values for user feedback.

| Sleep Pressure value | Verbal feedback |
| --- | --- |
| 0 - 40 | Low |
| 41 - 70 | Moderate |
| 71 - 100 | High |
| 100+ | Very high |

In addition, longer and more detailed feedback sentences may, for example, take into account the largest contributors toward sleep pressure. In a further exemplary embodiment, the feedback may take many forms, and different feedback can be given based on factors such as the time of day (such as before noon, afternoon, or evening), or the time elapsed after awakening from last night's sleep. For example, the "Afternoon" feedback can be given when the current "time from awake" value is greater than (24h - Sleep Need / 3) but less than ((24h-Sleep Need)/3)) * 2). In addition, one or more lifestyle factors from the given day can be considered when giving feedback on the Sleep Pressure status. An example may be as follows:

Table 15: Exemplary user feedback.

| Sleep Pressure value | Other rules | Feedback Sentence |
| --- | --- | --- |
| 0-40 | N.A. | You still have lots of energy available for activities. |
| 0-40 | Good sleep score from the previous night's sleep | You still have lots of energy available for activities due to your restorative sleep last night. |
| 41-60 | N.A. | There is some need for sleep, but not too much yet! |
| 41-60 | Vigorous exercise (Training Effect for the day over 3.0) | You still have moderate energy levels, even though your vigorous activity increased your recovery needs. |
| 61-75 | N.A. | The sleep pressure is piling up with the demands of the day. |
| 76-100 | N.A. | An earlier bedtime is suggested to ensure restorative sleep. Take the rest of the day easy. |
| 76-100 | Vigorous exercise (Training Effect for the day over 3.0) | Your need for restorative sleep is increased due to vigorous training. Take the rest of the day easy. |

Other potential feedback values may also be available. For example, it may of course be contemplated that a user's good sleep score from the previous night may be taken into account even when the user has a higher sleep pressure score, and exemplary feedback may include, for example, "You had lots of energy today due to your restorative sleep last night. You should go to bed soon if you want to have that happen tomorrow too!" Combinations of multiple rules in determining feedback may also be contemplated.

The user feedback in any of the above embodiments is meant as only an exemplary embodiment, and any combination of rules, factors, events, or data may be considered in providing feedback to the user.

The system and method according to the exemplary embodiments can be applied in many kinds of devices as would be understood by a person of ordinary skill in the art. For example, a wrist top device with a heart-rate transmitter (a fitness tracker, smartwatch, and the like), a mobile device such as a phone, tablet or the like, or another system having a CPU, memory and software therein (such as a fitness tracker dongle) may be used.

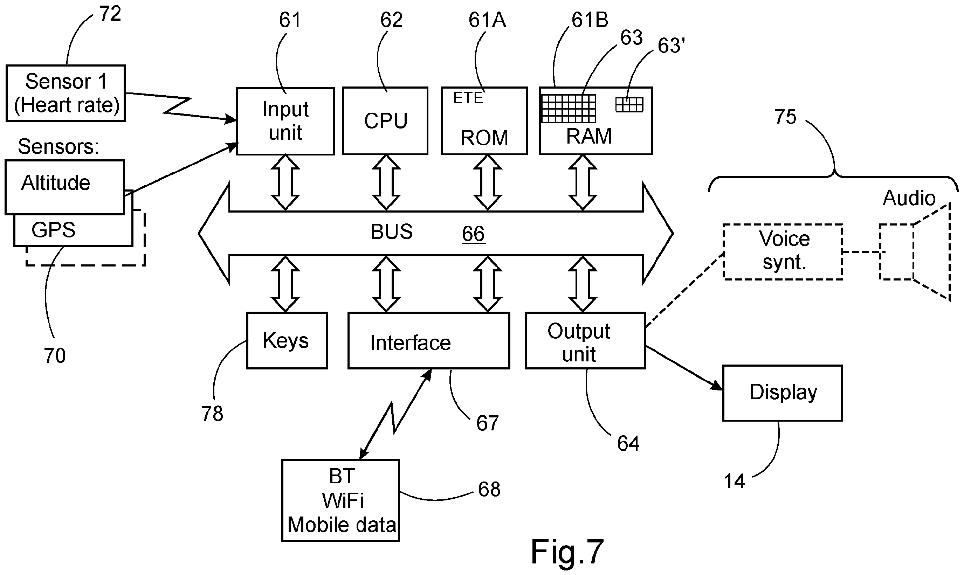

According to exemplary Fig. 7, the implementation may include an assembly built around a central processing unit (CPU) 62. A bus 66 may transmit data between the central unit 62 and the other units. The input unit 61, ROM memory 61A, RAM memory 61B including a dedicated memory, i.e. resident memory 63' for a register of the sleep need application and memory 63 for the host system, keys 78, PC connection 67, and output unit 64 may be connected to the bus. the term "resident memory" refers to specific RAM memory, which may be non-volatile memory or low-current memory. It keeps data long time, also after closing a process where it has been used, if desired.

The system may include a data logger which can be connected to cloud service, or other storage as would be understood by a person of ordinary skill in the art. The data logger may measure, for example, physiological response and/or external workload.

A heart rate sensor 72 and any sensor 70 registering external workload may be connected to the input unit 61, which may handle the sensor's data traffic to the bus 66. In some exemplary embodiments, the PC may be connected to a PC connection 67. The output device, for example a display 14 or the like, may be connected to output unit 64. In some embodiments, voice feedback may be created with the aid of, for example, a voice synthesizer and a loudspeaker 75, instead of, or in addition to the feedback on the display. As shown in Fig. 7, the sensor 70 which may measure external workload may be a GPS sensor, although this may be replaced by a number of sensors or different types of sensors, which may be used together to define the external work done by the user.

More specifically the apparatus presented in Figure 7 may have the following parts for determining sleep need parameters (like recommended bedtime):

- a heart rate sensor 72 configured to measure the heartbeat of the person, the heart rate signal being representative of the heartbeat of the user;
- optionally at least one sensor 70 to measure an external workload during an exercise, and
- a data processing unit 62 (CPU) operably coupled to the said sensors 72, 70, a memory 61A, 61B operably coupled to the data processing unit 62,
- the memory may be configured to save background information of a user, for example, background data including an earlier performance level, user characteristics, and the like.

The system may include a cloud service, or other storage as would be understood by a person of ordinary skill in the art. For example, in an exemplary embodiment wherein a smartphone or tablet is used, it may be contemplated to have data be stored in a persistent local log as well as in cloud storage, or data may be stored entirely locally if the user wishes to ensure that their data remains under their control, as desired. The cloud storage server may store, for example, physiological data regarding physiological resources, sleep need or pressure values, training load data or training load history, body stress levels or body stress history, user feedback, as well as the date the data was recorded, among other inputs. In an exemplary embodiment, the cloud storage server may receive inputs such as the physiological resource measurements and may analyze the data to calculate the body stress levels, training load data, or sleep period data. It may be contemplated that any of the described calculations may be performed by the cloud server and then a feedback response may be selected and presented to the user on the user's device. Additionally, the server may adjust the sleep need values and sleep pressure values based on the stored data. In an exemplary embodiment, the data may be stored numerically, or alternatively may be stored as a visual model, such as a line graph. The server may be configured to require a device-specific credential, such as a username and/or password in order to confirm the identity of the user and securely present the user with the data.

In some exemplary embodiments, the system may be connected to a PC via a variety of PC connections (such as USB, Bluetooth, Wi-Fi, etc.), allowing, for example, for direct uploading of data or more detailed analysis thereof. The output device, for example a display or the like, may be connected to an output unit configured to manage the output. In certain exemplary embodiments, the display may be on the same device as the system or may be on a different device. For example, it may be contemplated to have a fitness tracker that is paired with a smartphone and can send alerts to the smartphone to be displayed on the smartphone's screen. Another exemplary embodiment may send alerts to another device, such as a display screen of a device that the user is currently using, such as a laptop or desktop computer, to provide the user with information such as an approaching recommended bed time, or any other information that may be useful for the user to be aware of. In some embodiments, voice feedback may be created with the aid of, for example, a voice synthesizer and a loudspeaker, instead of, or in addition to, the feedback on the display. Such systems may likewise be on a device other than that performing the activity tracking, if desired. The sensor which may measure external workload may include any number of sensors, which may be used together to define the external work done by the user.

For example, according to an exemplary embodiment, an apparatus may have the following components used for sleep analysis. An exemplary apparatus may include a heart rate sensor configured to measure the heartbeat of the person, the heart rate signal being representative of the heartbeat of the user and may optionally include at least one sensor to measure an external workload during an exercise. The system may further include a data processing unit operably coupled to the sensors, and a memory unit operably coupled to the data processing unit. The memory unit may be configured to save background information of a user, for example, background data including an earlier sleep pressure level, user characteristics, and other relevant user information.

The apparatus may include dedicated software configured to execute the embodiments described in the present disclosure. For example, an exemplary embodiment of the sleep pressure application may make use of a small amount of RAM memory, approximately 100 - 400 bytes (x 8 bits), preferably 120 - 180 bytes. Generally, calculation may take place over a window of a plurality of days, e.g. 7 - 60 days. In a preferred embodiment, 20 - 40 days may be considered.

The foregoing description and accompanying figures illustrate the principles, preferred embodiments and modes of operation of the invention. However, the invention should not be construed as being limited to the particular embodiments discussed above. Additional variations of the embodiments discussed above will be appreciated by those skilled in the art (for example, features associated with certain configurations of the invention may instead be associated with any other configurations of the invention, as desired).

For example, as discussed previously, it may be contemplated in a number of exemplary embodiments to measure physiological data, and from this physiological data and other information, such as the user's sleep and awake periods, the time elapsed from a previous night's sleep, and a relationship between sleep pressure and the user's body resources, it may be contemplated to derive a user's estimated sleep pressure values. However, it may also be contemplated to make use of the above relationships in the opposite direction, such that sleep pressure may in turn be used in order to better characterize a user's apparent bodily resources. In one exemplary embodiment, it may be contemplated to generate and send a warning to the user when they are exercising very vigorously but have a high estimated sleep pressure value, warning the user not to strain themselves and advising them that they may have lower-than-expected body resource values due to lack of sleep. In another exemplary embodiment, a device that has access to estimated sleep need and sleep pressure data for a user but does not have access to certain physiological measurement sensors important for characterizing the user's body resources (such as, for example, a smartwatch with an accelerometer worn while sleeping that is equipped to measure the degree to which the user tosses and turns during sleep, or any other devices which can be used to measure sleep quality, but which does not have a heartbeat sensor) may derive estimates for the user's bodily resource values based on the user's estimated sleep need and sleep pressure, applying the above relationships in reverse.

Therefore, the above-described embodiments should be regarded as illustrative rather than restrictive. Accordingly, it should be appreciated that variations to those embodiments can be made by those skilled in the art without departing from the scope of the invention as defined by the following claims.

## Claims

1. A method for analyzing activity and providing feedback using an apparatus (80) with a heart rate sensor (72), a movement sensor (70), a processor (62), memory (61A, 62B) including a resident memory (63'), an output device (14) and software, by empirical modelling at least one of sleep need and sleep pressure using said apparatus, wherein the method comprises:

   determining, by the processor, a sleep need value based on user-specific attributes of a user and based on an activity of the user over a predetermined period, wherein the modelling includes separate models of personal sleep need from the baseline sleep need for at least one of following variables: stress and relaxation intensity, short-term training load, long-term training load, sleep score and absolute weekly training load, and wherein determining the sleep need value comprises:

   ; determining an age of the user, and determining a baseline sleep need value based on the age of the user; continuously measuring a plurality of physiological resource values of the user, wherein the physiological resource values comprise at least the two of sets of stress level data associated with and forming a body stress history, training load data associated with and forming a training history, and sleep period data comprising a plurality of sleep periods each having a period-specific sleep score; adjusting using the modelling the baseline sleep need value to the sleep need value based on at least two of the sets of the body stress history, the training history, and optionally each of the period-specific sleep scores;

   determining a current sleep pressure value based on the sleep need value, wherein sleep pressure depicts body's status regarding their need for sleep in real time, wherein determining the current sleep pressure value comprises:

determining a baseline sleep pressure value based on the sleep need value;

continuously monitoring a sleep and awake state of the user and analyzing one or more sleep periods and awake periods, and identifying a time elapsed since a last sleep period of the user;

determining a current body resource score of the user using measured user physiological data including at least heart rate variability data; and

adjusting using said modelling the baseline sleep pressure value to the current sleep pressure value based on the one or more sleep periods and awake periods, the current body resource score of the user, and the time elapsed since the last sleep period of the user;

calculating, based on the sleep need value and current sleep pressure values, at least one of a recommended bedtime and/or a recommended bedtime range; and

providing feedback according to a pre-set criterion.

2. The method according to claim 1, **characterized in that** continuously measuring the plurality of physiological resource values of the user comprises:

continuously measuring heart rate variability data of the user and movement data of the user; and

determining, from the heart rate variability data of the user and movement of the user, the plurality of physiological resource values.

3. The method according to claim 1 or 2, **characterized in that** the training load data further comprises at least one of training impulse data and excess post-exercise oxygen consumption data.

4. The method according to any of claims 1-3, **characterized in that** the modelling includes separate models for instant change of personal sleep pressure from an initial value based on age and history data, the models having each of the following variables: body resources and elapsed time.

5. The method according to any of claim 1-4, **characterized by** further steps of:

determining, based on location data of the user and time data, an expected outside light level, and determining, from the expected outside light level, a sleep pressure adjustment from light; and

adjusting the sleep pressure value by the sleep pressure adjustment from light.

6. The method according to any of claim 1-5, **characterized in that** adjusting the baseline sleep need value to the sleep need value based on the body stress history, the training history, and each of the period-specific sleep scores further comprises:

classifying training load as one of acute training load or chronic training load;

when the training load is an acute training load, comparing the training load to a cumulative training load metric, and when the training load is a chronic training load, comparing the training load to a periodic training load range, and obtaining a comparison result;

adjusting the sleep need value based on a classification of the training load and the comparison result.

7. The method according to any of claims 1-6, **characterized by** further step of: classifying each of the plurality of sleep periods of the sleep period data as one of: REM, light, deep, and awake.

8. The method according to any of claims 1-7, **characterized in that** continuously measuring the plurality of physiological resource values of the user further comprises:

measuring, with a motion sensor, an activity state of the user; and

determining, based on the activity state of the user, the plurality of sleep periods.

9. The method according to any claims 1-8, **characterized in that** automatically providing, to the user, the feedback dialogue comprises at least one of: displaying the feedback dialogue on a display, or playing the feedback dialogue via a speaker.

10. The method according to any of claims 1-9, **characterized in that** selecting the feedback dialogue to be displayed to the user further comprises:

determining, from a previous sleep need of the user, whether the sleep need value has decreased, increased, or remained the same as compared to the previous sleep need of the user; and

selecting a feedback based on a comparison with the previous sleep need of the user.

11. An apparatus for analyzing activity and providing feedback, comprising a processor (62), a memory (61A, 61B), a user input interface (78), an accelerometer, a gyroscope (70), a heart rate sensor (72), and at least one output module (14) software with empirical modelling sleep need and/or sleep pressure in function of physiological and movement variables,, the apparatus is configured to perform the steps of:

determining, by the processor (62), a sleep need value based on user-specific attributes of a user and based on an activity of the user over a predetermined period, wherein the modelling includes separate models of personal sleep need from the baseline sleep need for at least one of following variables: stress and relaxation intensity, short-term training load, long-term training load, sleep score and absolute weekly training load, and wherein determining the sleep need value comprises:

determining an age of the user, and determining a baseline sleep need value based on the age of the user; continuously measuring a plurality of physiological resource values of the user, wherein the physiological resource values comprise at least the set of: stress level data associated with and forming a body stress history, training load data associated with and forming a training history , and sleep period data comprising a plurality of sleep periods each having a period-specific sleep score; adjusting using said modelling the baseline sleep need value to the sleep need value based on the body stress history, the training history, and each of the period-specific sleep scores;

determining a current sleep pressure value based on the sleep need value, wherein sleep pressure depicts body's status regarding their need for sleep in real time, wherein determining the current sleep pressure value comprises:

determining a baseline sleep pressure value based on the sleep need value; continuously monitoring a sleep and awake state of the user and analyzing one or more sleep periods and awake periods, and identifying a time elapsed since a last sleep period of the user; determining a current body resource score of the user measuring using measured physiological data; and adjusting using said modelling the baseline sleep pressure value to the current sleep pressure value based on the one or more sleep periods and awake periods, the current body resource score of the user, and the time elapsed since the last sleep period of the user;

calculating, based on the sleep need value and current sleep pressure values, at least one of a recommended bedtime and a recommended bedtime range; selecting, from a set of feedback dialogues associated with predetermined sleep need and sleep pressure values, a feedback dialogue to be displayed to the user; and automatically providing, to the user feedback according to a pre-set criterion.

12. An apparatus according to claim 11, **characterized in that** the apparatus is implemented in one of following: heart rate monitor, fitness device, mobile phone, PDA device, wristop computer or personal computer having software for implementing said software means and hardware for execution of the software.

13. An apparatus according to claim 11 or 12, **characterized by** at least one sensor belonging to group a GPS-system, an accelerometer or by a power meter arranged to measure external workload.

14. An apparatus according to any of claim 11 - 13, **characterized in that** the apparatus has at least one output module and it is configured to give the feedback dialogue via said output module, wherein automatic provision of the feedback dialogue is provided at one of the set of: a predetermined time before the recommended bedtime and a predetermined time before a start of the recommended bedtime range.

15. A computer program product for analyzing activity and providing feedback embodied on a non-transitory computer-readable medium, the non-transitory computer-readable medium comprising program code that, when run on the apparatus of claims 11-14, causes a computer to perform the steps of:

determining, by a processor of the computer, a sleep need value based on user-specific attributes of a user and based on an activity of the user over a predetermined period of time, and empiric modelling, wherein the modelling includes separate models of personal sleep need from the baseline sleep need for at least one of following variables: stress and relaxation intensity, short-term training load, long-term training load, sleep score and absolute weekly training load, and wherein determining the sleep need value comprises:

determining an age of the user, and determining a baseline sleep need value based on the age of the user; continuously measuring a plurality of physiological resource values of the user, wherein the physiological resource values comprise at least the set of: stress level data associated with and forming a body stress history, training load data associated with and forming a training history, and sleep period data comprising a plurality of sleep periods each having a period-specific sleep score; adjusting using said meddling the baseline sleep need value to the sleep need value based on the body stress history, the training history, and each of the period-specific sleep scores;

determining a current sleep pressure value based on the sleep need value, wherein sleep pressure depicts body's status regarding their need for sleep in real time, wherein determining the current sleep pressure value comprises:

determining a baseline sleep pressure value based on the sleep need value; continuously monitoring a sleep and awake state of the user and analyzing one or more sleep periods and awake periods, and identifying a time elapsed since a last sleep period of the user; measuring user physiological data and determining a current body resource score of the user; and adjusting using said modelling the baseline sleep pressure value to the current sleep pressure value based on the one or more sleep periods and awake periods, the current body resource score of the user, and the time elapsed since the last sleep period of the user;

calculating, based on the sleep need value and current sleep pressure values, at least one of a recommended bedtime and a recommended bedtime range; selecting, from a set of feedback dialogues associated with predetermined sleep need and sleep pressure values, a feedback dialogue to be displayed to the user; and automatically providing, to the user, the feedback dialogue, wherein automatic provision of the feedback dialogue is provided at one of the sets of: a predetermined time before the recommended bedtime and a predetermined time before a start of the recommended bedtime range.

## Patentansprüche

1. Verfahren zur Analyse von Aktivitäten und Bereitstellen von Rückmeldungen mithilfe einer Vorrichtung (80) mit einem Herzfrequenzsensor (72), einem Bewegungssensor (70), einem Prozessor (62), Speicher (61A, 62B) einschließlich eines residenten Speichers (63'), einer Ausgabevorrichtung (14) und Software,
durch empirisches Modellieren von mindestens entweder dem Schlafbedürfnis oder dem Schlafdruck mithilfe der Vorrichtung, wobei das Verfahren die folgenden Schritte umfasst:

Bestimmen eines Schlafbedürfniswerts auf Basis der benutzerspezifischen Attribute eines Nutzers durch den Prozessor und auf der Basis einer Aktivität des Nutzers über einen festgelegten Zeitraum, wobei die Modellierung separate Modelle des persönlichen Schlafbedürfnisses aus dem Basisschlafbedürfnis für mindestens eine der folgenden Variablen einschließt: Stress- und Erholungsintensität, kurzfristige Trainingsbelastung, langfristige Trainingsbelastung, Schlafpunktzahl und absolute wöchentliche Trainingsbelastung, und wobei die Bestimmung des Schlafbedürfniswerts Folgendes umfasst:

Bestimmen des Alters des Nutzers und Bestimmen eines Basisschlafbdürfniswerts auf der Basis des Alters des Nutzers; kontinuierliches Messen einer Vielzahl von physiologischen Ressourcenwerten des Nutzers, wobei die physiologischen Ressourcenwerte mindestens die zwei Sätze von Stressniveaudaten umfassen, die mit der Körperstreßhistorie assoziiert sind und diese bilden, den Trainingsbelastungsdaten, die mit der Trainingshistorie assoziiert sind und diese bilden und den Schlafzeitraumdaten, umfassend eine Vielzahl von Schlafzeiträumen, die jeweils einen zeitraumspezifischen Schlafpunktwert aufweisen; Anpassung unter Verwendung des Basischlafbedürfniswerts an den Schlafbedürfniswert auf der Basis von mindestens zwei der Sätze an Körperstreßhistorie, der Trainingshistorie und optional jedem der zeitraumspezifischen Schlafwerte;

Bestimmen eines aktuellen Schlafdruckwerts auf der Basis des Schlafbedürfniswerts, wobei der Schlaf druck den Körperstatus abbildet in Bezug auf dessen Bedürfnis an Schlaf in Echtzeit, wobei die Bestimmung des aktuellen Schlafdruckwerts Folgendes umfasst:

Bestimmen des Basisschlafdruckwerts auf der Basis des Schlafbedürfniswerts;
kontinuierliches Überwachen eines Schlaf- und Wachzustands des Nutzers und Analyse eines oder mehrerer Schlafzeiträume und Wachzeiträume, und Identifizieren einer verstrichenen Zeit seit des letzten Schlafzeirams des Nutzers;
Bestimmen eines aktuellen Körperressourcenwerts des Nutzers unter Verwendung gemessener physiologischer Daten des Nutzers einschließlich mindestens der Herzfrequenzvariabilitätsdaten; und
Anpassen des Basisschlafdruckwertes unter Verwendung der Modellierung an den aktuellen Schlafdruckwert auf der Basis des einen oder der mehreren Schlafzeiträume und Wachzeiträume, dem aktuellen Körperressourcenpunktwert des Nutzers und der seit dem letzten Schlafzeitraum des Nutzers verstrichenen Zeit; Berechnen mindestens entweder eine empfohlene Schlafenszeit bzw. eines empfohlenen Schlafenszeitbereichs auf der Basis des Schlafbedürfniswertes und der aktuellen Schlafdruckwerte; und

Bereitstellung einer Rückmeldung entsprechend eines voreingestellten Kriteriums.

2. Verfahren nach Anspruch 1, **dadurch gekennzeichnet, dass** die kontinuierliche Messung der Vielzahl von physiologischen Ressourenwerte des Nutzers Folgendes umfasst:

kontinuierliches Messen der Herzfrequenzvariabilitätsdaten des Nutzers und der Bewegungsdaten des Nutzers; und
Bestimmen der Vielzahl von physiologischen Ressourcenwerte aus den Herzfrequenzvariabilitätsdaten des Nutzers und den Bewegungsdaten des Nutzers.

3. Das Verfahren nach Anspruch 1 oder 2, **dadurch gekennzeichnet, dass** die Trainingsbelastungsdaten ferner mindestens entweder die Trainingsimpulsdaten oder übermäßige Sauerstoffverbrauchsdaten nach einer Übung umfasst.

4. Verfahren nach einem der Ansprüche 1-3, **dadurch gekennzeichnet, dass** die Modellierung separate Modelle für einen sofortigen Wechsel des persönlichen Schlafdrucks von einem Anfangswert auf der Basis von Alter und Historiendaten einschließt, wobei die Modelle jeweils die folgenden Variablen aufweisen: Körperressourcen und verstrichene Zeit.

5. Verfahren nach einem der Ansprüche 1-4, **gekennzeichnet durch** die weiteren Schritte von:

Bestimmen, auf der Basis der Ortsdaten des Nutzers und der Zeitdaten, einer erwarteten Außenlichtmenge und Bestimmen, aus der erwarteten Außenlichtmenge, einer Schlafdruckanpassung durch das Licht; und
Anpassen des Schlafdruckwerts um die Schlafdruckanpassung durch das Licht.

6. Verfahren nach einem der Ansprüche 1-5, **dadurch gekennzeichnet, dass** die Anpassung des Basischlafbedürfniswerts an den Schlafbedürfniswert auf der Basis der Körperstreßhistorie, der Trainingshistorie und jedem der zeitraumspezifischen Schlafwerte ferner Folgendes umfasst:

Kategorisieren der Trainingsbelastung als eine akute Trainingsbelastung oder eine chronische Trainingsbelastung;
im Falle einer akuten Trainingsbelastung, Vergleich der Trainingsbelastung mit einer kumulativen Trainingsbelastungsmetrik und dann, wenn die Trainingsbelastung eine chronische Trainingsbelastung ist, Vergleich der Trainingsbelastung mit einem regelmäßigen Trainingsbelastungbereich und Erhalt eines Vergleichsergebnisses;
Anpassung des Schlafbedürfniswerts auf der Basis einer Kategorisierung der Trainingsbelastung und des Vergleichsergebnisses.

7. Verfahren nach einem der Ansprüche 1-6, **gekennzeichnet durch** den weiteren Schritt von: Kategorisieren jeder der Vielzahl von Schlafzeiträumen der Schlafzeitraumdaten als entweder: REM, leicht, tief oder wach.

8. Verfahren nach einem der Ansprüche 1-7, **dadurch gekennzeichnet, dass** die kontinuierliche Messung der Vielzahl von physiologischen Ressourenwerte des Nutzers ferner Folgendes umfasst:

Messen eines Aktivitätszustands des Nutzers mit einem Bewegungssensor; und
Bestimmen der Vielzahl der Schlafzeiträume auf der Basis des Aktivitätszustands des Nutzers.

9. Verfahren nach einem der Ansprüche 1-8, **dadurch gekennzeichnet, dass** die automatische Bereitstellung des Rückmeldedialogs an den Nutzer mindestens eines der Folgenden umfasst: Anzeige des Rückmeldedialogs auf einer Anzeige oder Abspielen des Rückmeldedialogs über einen Lautsprecher.

10. Verfahren nach einem der Ansprüche 1-9, **dadurch gekennzeichnet, dass** die Auswahl das dem Nutzer anzuzeigenden Rückmeldedialogs ferner Folgendes umfasst:

Bestimmen, aus einem früheren Schlafbedürfnis des Nutzers, ob der Schlafbedürfniswert sich verringert oder erhöht hat oder gleich geblieben ist im Vergleich zu dem vorherigen Schlafbedürfnis des Nutzers; und
Auswahl einer Rückmeldung auf der Basis eines Vergleichs mit dem vorherigen Schlafbedürfnis des Nutzers.

11. Vorrichtung zur Analyse von Aktivität und Bereitstellung von Rückmeldungen, umfassend einen Prozessor (62), einen Speicher (61A, 61B), eine Nutzereingabeschnittstelle (78), einen Beschleunigungsmesser, ein Gyroskop (70), einen Herzfrequenzsensor (72) und mindestens ein Ausgabemodul (14) Software mit empirischer Modellierung des Schlafbedürfnis bzw. Schlafdrucks als eine Funktion von physiologischen und Bewegungsvariablen, wobei die Vorrichtung ausgelegt ist, um die folgenden Schritte auszuführen:

Bestimmen eines Schlafbedürfniswerts auf Basis der benutzerspezifischen Attribute eines Nutzers durch den Prozessor (62) und auf der Basis einer Aktivität des Nutzers über einen festgelegten Zeitraum, wobei die Modellierung separate Modelle des persönlichen Schlafbedürfnisses aus dem Basisschlafbedüfrnis für mindestens eine der folgenden Variablen einschließt: Stress- und Erholungsintensität, kurzfristige Trainingsbelastung, langfristige Trainingsbelastung, Schlafpunktzahl und absolute wöchentliche Trainingsbelastung, und wobei die Bestimmung des Schlafbedürfniswerts Folgendes umfasst:

Bestimmen des Alters des Nutzers und Bestimmen eines Basisschlafbdürfniswerts auf der Basis des Alters des Nutzers;
kontinuierliches Messen einer Vielzahl von physiologischen Ressourcenwerten des Nutzers, wobei die physiologischen Ressourcenwerte mindestens den Satz von Stressniveaudaten umfassen, die mit der Körperstreßhistorie assoziiert sind und diese bilden, den Trainingsbelastungsdaten, die mit der Trainingshistorie assoziiert sind und diese bilden und den Schlafzeitraumdaten, umfassend eine Vielzahl von Schlafzeiträumen, die jeweils einen zeitraumspezifischen Schlafpunktwert aufweisen;
Anpassung unter Verwendung der Modellierung des Basischlafbedürfniswerts an den Schlafbedürfniswert auf der Basis der Körperstreßhistorie, der Trainingshistorie und optional jedem der zeitraumspezifischen Schlafpunktwerte;

Bestimmen eines aktuellen Schlafdruckwerts auf der Basis des Schlafbedürfniswerts, wobei der Schlaf druck den Körperstatus abbildet in Bezug auf dessen Bedürfnis an Schlaf in Echtzeit, wobei die Bestimmung des aktuellen Schlafdruckwerts Folgendes umfasst:

Bestimmen des Basisschlafdruckwerts auf der Basis des Schlafbedürfniswerts;
kontinuierliches Überwachen eines Schlaf- und Wachzustands des Nutzers und Analyse eines oder mehrerer Schlafzeiträume und Wachzeiträume, und Identifizieren einer verstrichenen Zeit seit dem letzten Schlafzeitraum des Nutzers;
Bestimmen eines aktuellen Körperressourcenpunktwerts des Nutzers unter Verwendung gemessener physiologischer Daten; und
Anpassen des Basisschlafdruckwertes unter Verwendung der Modellierung an den aktuellen Schlafdruckwert auf der Basis des einen oder der mehreren Schlafzeiträume und Wachzeiträume, dem aktuellen Körperressourcenpunktwert des Nutzers und der seit dem letzten Schlafzeitraum des Nutzers verstrichenen Zeit;

Berechnen mindestens entweder einer empfohlenen Schlafenszeit bzw. eines empfohlenen Schlafenszeitbereichs auf der Basis des Schlafbedürfniswertes und der aktuellen Schlafdruckwerte;

Auswahl eines dem Nutzer anzuzeigenden Rückmeldedialogs aus einem Satz von Rückmeldedialogen, die mit den vorbestimmten Schlafbedürfnis- und Schlafdruckwerten assoziiert sind; und automatische Bereitstellung einer Rückmeldung an den Nutzer entsprechend eines voreingestellten Kriteriums.

12. Vorrichtung nach Anspruch 11, **dadurch gekennzeichnet, dass** die Vorrichtung in einer der folgenden Vorrichtungen implementiert ist: Herzfrequenzmesser, Fitnessgerät, Mobiltelefon, PDA-Gerät, Armbandcomputer oder Personalcomputer mit Software zum Implementieren der Softwaremittel und Hardware zur Ausführung der Software.

13. Vorrichtung nach Anspruch 11 oder 12, **gekennzeichnet durch** mindestens einen Sensor, der zur Gruppe der GPS-Systeme gehört, eines Beschleunigungsmessers oder eines Leistungsmessers, der zur Messung der externen Arbeitslast angeordnet ist.

14. Vorrichtung nach einem der Ansprüche 11-13, **dadurch gekennzeichnet, dass** die Vorrichtung mindestens ein Ausgabemodul aufweist und diese konfiguriert ist, um den Rückmeldedialog über das Ausgabemodul zu übermitteln, wobei eine automatische Bereitstellung des Rückmeldedialogs an einem Punkt des folgenden Satzes erfolgt: einem vorbestimmten Zeitpunkt vor der empfohlenen Schlafenszeit und einem vorbestimmten Zeitpunkt vor dem Beginn eines empfohlenen Schlafenszeitbereichs.

15. Computerprogrammprodukt zur Analyse von Aktivität und Bereitstellung von Rückmeldungen, die auf einem nicht-flüchtigen computerlesbaren Medium ausgeführt sind, das nicht-flüchtige computerlesbare Medium umfassend einen Programmcode, der, bei Ausführung auf einer Vorrichtung gemäß Anspruch 11-14, einen Computer dazu veranlasst, die folgenden Schritte auszuführen:

Bestimmen eines Schlafbedürfniswerts auf Basis der benutzerspezifischen Attribute eines Nutzers durch den Prozessor und auf der Basis einer Aktivität des Nutzers über einen festgelegten Zeitraum, und empirische Modellierung, wobei die Modellierung separate Modelle des persönlichen Schlafbedürfnisses aus dem Basisschlafbedüfrnis für mindestens eine der folgenden Variablen einschließt: Stress- und Erholungsintensität, kurzfristige Trainingsbelastung, langfristige Trainingsbelastung, Schlafpunktzahl und absolute wöchentliche Trainingsbelastung, und wobei die Bestimmung des Schlafbedürfniswerts Folgendes umfasst:

Bestimmen des Alters des Nutzers und Bestimmen eines Basisschlafbdürfniswerts auf der Basis des Alters des Nutzers;
kontinuierliches Messen einer Vielzahl von physiologischen Ressourcenwerten des Nutzers, wobei die physiologischen Ressourcenwerte mindestens den Satz von Stressniveaudaten umfassen, die mit der Körperstreßhistorie assoziiert sind und diese bilden, den Trainingsbelastungsdaten, die mit der Trainingshistorie assoziiert sind und diese bilden und den Schlafzeitraumdaten, umfassend eine Vielzahl von Schlafzeiträumen, die jeweils einen zeitraumspezifischen Schlafpunktwert aufweisen;
Anpassen unter Verwendung der Mittelung des Basisschlafbedürfniswerts an den Schlafbedürfniswert auf der Basis der Körperstreßhistorie, der Trainingshistorie und jedem der zeitraumspezifischen Schlafpunktwerte;

Bestimmen eines aktuellen Schlafdruckwerts auf der Basis des Schlafbedürfniswerts, wobei der Schlaf druck den Körperstatus abbildet in Bezug auf dessen Bedürfnis an Schlaf in Echtzeit, wobei die Bestimmung des aktuellen Schlafdruckwerts Folgendes umfasst:

Bestimmen des Basisschlafdruckwerts auf der Basis des Schlafbedürfniswerts;
kontinuierliches Überwachen eines Schlaf- und Wachzustands des Nutzers und Analyse eines oder mehrerer Schlafzeiträume und Wachzeiträume, und Identifizieren einer verstrichenen Zeit seit dem letzten Schlafzeitraum des Nutzers;
Messen der physiologischen Daten des Nutzers und Bestimmen eines aktuellen Körperressourcenpunktwerts des Nutzers; und
Anpassen des Basisschlafdruckwerts unter Verwendung der Modellierung an den aktuellen Schlafdruckwert auf der Basis des einen oder der mehreren Schlafzeiträume und Wachzeiträume, dem aktuellen Körperressourcenpunktwert des Nutzers und der seit dem letzten Schlafzeitraum des Nutzers verstrichenen Zeit;

Berechnen mindestens entweder einer empfohlenen Schlafenszeit bzw. eines empfohlenen Schlafenszeitbereichs auf der Basis des Schlafbedürfniswertes und der aktuellen Schlafdruckwerte;
Auswahl eines dem Nutzer anzuzeigenden Rückmeldedialogs aus einem Satz von Rückmeldedialogen, die mit den vorbestimmten Schlafbedürfnis- und Schlafdruckwerten assoziiert sind; und
automatische Bereitstellung des Rückmeldedialogs an einem Punkt des folgenden Satzes erfolgt: einem vorbestimmten Zeitpunkt vor der empfohlenen Schlafenszeit und einem vorbestimmten Zeitpunkt vor dem Beginn eines empfohlenen Schlafenszeitbereichs.

**Revendications**

1. Procédé permettant d'analyser l'activité et de fournir un retour d'information à l'aide d'un appareil (80) équipé d'un capteur de fréquence cardiaque (72), d'un capteur de mouvement (70), d'un processeur (62), d'un système de mémoire (61A, 62B) comprenant une mémoire résidente (63'), d'un dispositif de sortie (14) et d'un logiciel, en modélisant de manière empirique le besoin en sommeil ou la pression du sommeil à l'aide dudit appareil, ledit procédé comprenant :

la détermination, par le processeur, d'une valeur de besoin en sommeil basée sur des attributs propres à un utilisateur et sur une activité de l'utilisateur au cours d'une période prédéterminée, la modélisation comprenant des modèles distincts de besoin en sommeil personnel reposant sur le besoin en sommeil de référence pour au moins l'une des variables suivantes : intensité de l'effort et de la relaxation, charge d'entraînement à court terme, charge d'entraînement à long terme, score de sommeil et charge d'entraînement hebdomadaire absolue, la détermination de la valeur de besoin en sommeil comprenant :

la détermination de l'âge de l'utilisateur, et la détermination d'une valeur de besoin en sommeil de référence en fonction de l'âge de l'utilisateur ;
la mesure continue de plusieurs valeurs liées aux ressources physiologiques de l'utilisateur, les valeurs liées aux ressources physiologiques comprenant au moins deux ensembles de données parmi les suivants : données sur le niveau d'effort associées à l'historique d'effort physique et formant cet historique, données sur la charge d'entraînement associées à un historique d'entraînement et formant cet historique, et données sur les périodes de sommeil comprenant plusieurs périodes de sommeil, chacune assortie d'un score de sommeil propre ;
l'ajustement, à l'aide de la modélisation, de la valeur de besoin en sommeil de référence à la valeur de besoin en sommeil en fonction d'au moins deux ensembles de données parmi les suivants : historique d'effort physique, historique d'entraînement et, en option, chacun des scores de sommeil propres à une période ;

la détermination d'une valeur actuelle de pression du sommeil en fonction de la valeur de besoin en sommeil, la pression du sommeil représentant l'état du corps concernant son besoin en sommeil en temps réel, la détermination de la valeur actuelle de la pression du sommeil comprenant :

la détermination d'une valeur de pression du sommeil de référence en fonction de la valeur de besoin en sommeil ;
la surveillance continue de l'état de sommeil et d'éveil de l'utilisateur et l'analyse d'une ou de plusieurs périodes de sommeil et d'éveil, et l'identification du temps écoulé depuis la dernière période de sommeil de l'utilisateur ;
la détermination du score actuel de ressources corporelles de l'utilisateur à l'aide des données physiologiques mesurées de l'utilisateur, y compris au moins les données sur la variabilité de la fréquence cardiaque ; et
l'ajustement, à l'aide de ladite modélisation, de la valeur de référence de pression du sommeil à la valeur actuelle de pression du sommeil en fonction de la période ou des périodes de sommeil et d'éveil, du score actuel de ressources corporelles de l'utilisateur et du temps écoulé depuis la dernière période de sommeil de l'utilisateur ;

le calcul, sur la base de la valeur de besoin en sommeil et des valeurs actuelles de pression du sommeil, d'au moins l'un des éléments suivants : une heure de coucher recommandée et une plage horaire de coucher recommandée ; et
la transmission d'un retour d'information en fonction d'un critère présélectionné.

2. Procédé conformément à la revendication 1, **caractérisé en ce que** la mesure continue de plusieurs valeurs liées aux ressources physiologiques de l'utilisateur comprend :

la mesure continue des données sur la variabilité de la fréquence cardiaque de l'utilisateur et des données sur le mouvement de l'utilisateur ; et

la détermination, à partir des données sur la variabilité de la fréquence cardiaque de l'utilisateur et du mouvement de l'utilisateur, de plusieurs valeurs liées aux ressources physiologiques.

3. Procédé conformément à la revendication 1 ou 2, **caractérisé en ce que** les données sur la charge d'entraînement comprennent également au moins l'un des éléments suivants : des données sur les stimuli d'entraînement et des données sur la surconsommation d'oxygène après l'exercice.

4. Procédé conformément à l'une quelconque des revendications 1 à 3, **caractérisé en ce que** la modélisation comprend des modèles distincts pour un changement instantané de pression du sommeil personnel à partir d'une valeur initiale basée sur l'âge et l'historique des données, les modèles comprenant chacune des variables suivantes : ressources corporelles et temps écoulé.

5. Procédé conformément à l'une quelconque des revendications 1 à 4, **caractérisé par** les autres étapes suivantes :

la détermination, en fonction des données de localisation de l'utilisateur et de données temporelles, d'un niveau de luminosité extérieure attendu, et la détermination, à partir du niveau de luminosité extérieure attendu, d'un ajustement de la pression du sommeil par rapport à la luminosité ; et

l'ajustement de la valeur de pression du sommeil grâce à l'ajustement de la pression du sommeil par rapport à la luminosité.

6. Procédé conformément à l'une quelconque des revendications 1 à 5, **caractérisé en ce que** l'ajustement de la valeur de besoin en sommeil de référence à la valeur de besoin en sommeil en fonction de l'historique d'effort physique, de l'historique d'entraînement et de chacun des scores de sommeil propres à une période comprend également :

la classification de la charge d'entraînement en tant que charge d'entraînement extrême ou charge d'entraînement chronique ;

lorsque la charge d'entraînement est extrême, la comparaison de la charge d'entraînement à un indicateur de charge d'entraînement cumulée, et, lorsque la charge d'entraînement est chronique, la comparaison de la charge d'entraînement à une fourchette de charges d'entraînement périodique, et l'obtention d'un résultat de comparaison ;

l'ajustement de la valeur de besoin en sommeil en fonction d'une classification de la charge d'entraînement et du résultat de comparaison.

7. Procédé conformément à l'une quelconque des revendications 1 à 6, **caractérisé par** une autre étape : la classification de chacune des périodes de sommeil parmi les données sur les périodes de sommeil dans les catégories suivantes : sommeil paradoxal, sommeil léger, sommeil profond et éveil.

8. Procédé conformément à l'une quelconque des revendications 1 à 7, **caractérisé en ce que** la mesure continue de plusieurs valeurs liées aux ressources physiologiques de l'utilisateur comprend également :

la mesure, à l'aide d'un capteur de mouvement, d'un état d'activité de l'utilisateur ; et

la détermination, en fonction de l'état d'activité de l'utilisateur, de plusieurs périodes de sommeil.

9. Procédé conformément à l'une quelconque des revendications 1 à 8, **caractérisé en ce que** la transmission automatique, à l'utilisateur, de la boîte de dialogue de retour d'information comprend au moins l'une des actions suivantes : l'affichage de la boîte de dialogue de retour d'information sur un écran ou la lecture de la boîte de dialogue de retour d'information via un haut-parleur.

10. Procédé conformément à l'une quelconque des revendications 1 à 9, **caractérisé en ce que** la sélection de la boîte de dialogue de retour d'information à présenter à l'utilisateur comprend également :

la détermination, à partir du besoin en sommeil précédent de l'utilisateur, de la variation de la valeur du besoin en sommeil, à savoir si elle a diminué, si elle a augmenté ou si elle est restée la même, par rapport au précédent besoin en sommeil de l'utilisateur ; et

la sélection d'un retour d'information en fonction d'une comparaison avec le besoin en sommeil précédent de l'utilisateur.

11. Appareil pour l'analyse de l'activité et la transmission d'un retour d'information, comprenant un processeur (62), un système de mémoire (61A, 61B), une interface permettant à l'utilisateur de saisir des données (78), un accéléromètre, un gyroscope (70), un capteur de fréquence cardiaque (72) et au moins un module logiciel de sortie (14) avec modélisation empirique du besoin en sommeil et/ou de la pression du sommeil en fonction de variables physiologiques et de mouvement, l'appareil étant configuré pour exécuter les étapes suivantes :

la détermination, par le processeur (62), d'une valeur de besoin en sommeil basée sur des attributs propres à un utilisateur et sur une activité de l'utilisateur au cours d'une période prédéterminée, la modélisation comprenant des modèles distincts de besoin en sommeil personnel reposant sur le besoin en sommeil de référence pour au moins l'une des variables suivantes : intensité de l'effort et de la relaxation, charge d'entraînement à court terme, charge d'entraînement à long terme, score de sommeil et charge d'entraînement hebdomadaire absolue, la détermination de la valeur de besoin en sommeil comprenant :

la détermination de l'âge de l'utilisateur, et la détermination d'une valeur de besoin en sommeil de référence en fonction de l'âge de l'utilisateur ;
la mesure continue de plusieurs valeurs liées aux ressources physiologiques de l'utilisateur, les valeurs liées aux ressources physiologiques comprenant au moins un ensemble de données parmi les suivants : données sur le niveau d'effort associées à l'historique d'effort physique et formant cet historique, données sur la charge d'entraînement associées à un historique d'entraînement et formant cet historique, et données sur les périodes de sommeil comprenant plusieurs périodes de sommeil, chacune assortie d'un score de sommeil propre ;
l'ajustement, à l'aide de ladite modélisation, de la valeur de besoin en sommeil de référence à la valeur de besoin en sommeil en fonction de l'historique d'effort physique, de l'historique d'entraînement et de chacun des scores de sommeil propres à une période ;

la détermination d'une valeur actuelle de pression du sommeil en fonction de la valeur de besoin en sommeil, la pression du sommeil représentant l'état du corps concernant son besoin en sommeil en temps réel, la détermination de la valeur actuelle de la pression du sommeil comprenant :

la détermination d'une valeur de pression du sommeil de référence en fonction de la valeur de besoin en sommeil ;
la surveillance continue de l'état de sommeil et d'éveil de l'utilisateur et l'analyse d'une ou de plusieurs périodes de sommeil et d'éveil, et l'identification du temps écoulé depuis la dernière période de sommeil de l'utilisateur ;
la détermination du score actuel de ressources corporelles de l'utilisateur à l'aide de données physiologiques mesurées ; et
l'ajustement, à l'aide de ladite modélisation, de la valeur de référence de pression du sommeil à la valeur actuelle de pression du sommeil en fonction de la période ou des périodes de sommeil et d'éveil, du score actuel de ressources corporelles de l'utilisateur et du temps écoulé depuis la dernière période de sommeil de l'utilisateur ;

le calcul, sur la base de la valeur de besoin en sommeil et des valeurs actuelles de pression du sommeil, d'au moins l'un des éléments suivants : une heure de coucher recommandée et une plage horaire de coucher recommandée ;
la sélection, à partir d'un ensemble de boîtes de dialogue de retour d'information associées à des valeurs de besoin en sommeil et de pression du sommeil prédéterminées, d'une boîte de dialogue de retour d'information à présenter à l'utilisateur ; et
la transmission automatique à l'utilisateur d'un retour d'information en fonction d'un critère présélectionné.

12. Appareil conformément à la revendication 11, **caractérisé en ce que** l'appareil est mis en oeuvre dans l'un des dispositifs suivants : cardiofréquencemètre, dispositif de conditionnement physique, téléphone portable, assistant numérique personnel, montre connectée ou PC doté d'un logiciel permettant l'installation dudit logiciel et du matériel permettant l'exécution du logiciel.

13. Appareil conformément à la revendication 11 ou 12, **caractérisé par** au moins un capteur appartenant au groupe suivant : un système GPS, un accéléromètre ou un wattmètre prévu pour mesurer une charge de travail externe.

**14.** Appareil conformément à l'une quelconque des revendications 11 à 13, **caractérisé en ce que** l'appareil comporte au moins un module de sortie et est configuré pour transmettre la boîte de dialogue d'information en retour via ledit module de sortie, la transmission automatique de la boîte de dialogue d'information en retour ayant lieu à l'un des moments suivants : une heure prédéterminée avant l'heure de coucher recommandée et une heure prédéterminée avant le début de la plage horaire de coucher recommandée.

**15.** Programme informatique pour l'analyse de l'activité et la transmission d'information en retour incorporé à un support non transitoire lisible par ordinateur, ledit support non transitoire lisible par ordinateur comprenant du code de programmation qui, lorsqu'il est exécuté sur l'appareil visé dans les revendications 11 à 14, lance l'exécution par un ordinateur des étapes suivantes :

la détermination, par un processeur de l'ordinateur, d'une valeur de besoin en sommeil basée sur des attributs propres à un utilisateur et sur une activité de l'utilisateur au cours d'une période de temps prédéterminée, et la modélisation empirique, la modélisation comprenant des modèles distincts de besoin en sommeil personnel reposant sur le besoin en sommeil de référence pour au moins l'une des variables suivantes : intensité de l'effort et de la relaxation, charge d'entraînement à court terme, charge d'entraînement à long terme, score de sommeil et charge d'entraînement hebdomadaire absolue, la détermination de la valeur de besoin en sommeil comprenant :

la détermination de l'âge de l'utilisateur, et la détermination d'une valeur de besoin en sommeil de référence en fonction de l'âge de l'utilisateur ;
la mesure continue de plusieurs valeurs liées aux ressources physiologiques de l'utilisateur, les valeurs liées aux ressources physiologiques comprenant au moins un ensemble de données parmi les suivants : données sur le niveau d'effort associées à l'historique d'effort physique et formant cet historique, données sur la charge d'entraînement associées à un historique d'entraînement et formant cet historique, et données sur les périodes de sommeil comprenant plusieurs périodes de sommeil, chacune assortie d'un score de sommeil propre ;
l'ajustement, à l'aide de ladite modélisation, de la valeur de besoin en sommeil de référence à la valeur de besoin en sommeil en fonction de l'historique d'effort physique, de l'historique d'entraînement et de chacun des scores de sommeil propres à une période ;

la détermination d'une valeur actuelle de pression du sommeil en fonction de la valeur de besoin en sommeil, la pression du sommeil représentant l'état du corps concernant son besoin en sommeil en temps réel, la détermination de la valeur actuelle de la pression du sommeil comprenant :

la détermination d'une valeur de pression du sommeil de référence en fonction de la valeur de besoin en sommeil ;
la surveillance continue de l'état de sommeil et d'éveil de l'utilisateur et l'analyse d'une ou de plusieurs périodes de sommeil et d'éveil, et l'identification du temps écoulé depuis la dernière période de sommeil de l'utilisateur ;
la mesure des données physiologiques de l'utilisateur et la détermination du score actuel de ressources corporelles de l'utilisateur ; et
l'ajustement, à l'aide de ladite modélisation, de la valeur de référence de pression du sommeil à la valeur actuelle de pression du sommeil en fonction de la période ou des périodes de sommeil et d'éveil, du score actuel de ressources corporelles de l'utilisateur et du temps écoulé depuis la dernière période de sommeil de l'utilisateur ;

le calcul, sur la base de la valeur de besoin en sommeil et des valeurs actuelles de pression du sommeil, d'au moins l'un des éléments suivants : une heure de coucher recommandée et une plage horaire de coucher recommandée ;
la sélection, à partir d'un ensemble de boîtes de dialogue de retour d'information associées à des valeurs de besoin en sommeil et de pression du sommeil prédéterminées, d'une boîte de dialogue de retour d'information à présenter à l'utilisateur ; et
la transmission automatique, à l'utilisateur, de la boîte de dialogue d'information en retour, ladite transmission automatique de la boîte de dialogue d'information en retour ayant lieu à l'un des moments suivants : une heure prédéterminée avant l'heure de coucher recommandée et une heure prédéterminée avant le début de la plage horaire de coucher recommandée.

100

| 106 | Input user background parameters, at least age |
| 108 | Measure physiological data (HRV and ACC) continuously as inputs |
| 110 | Analyze body stress from HRV and ACC preferably 24h |
| 112 | Store body stress history from last 7 days |
| 114 | Measure physiological data (HRV and ACC) from training sessions |
| 116 | Store training history and analyze training load metrics (short-term load, long-term load, and short to long-term load ratio) |
| 118 | Detect sleep periods from HRV and AC and analyze sleep score |
| 120 | Store Sleep Score from preceding sleep period |
| 122 | Anaylze and provide feedback on the Sleep Need based on age, training load history, body stress history, and sleep history |

102

Analysis process for The Sleep Need, i.e. Recommended sleep duration.

| 124 | Input the Sleep Need |
| 126 | Detect sleep and awake periods continuously |
| 128 | Analyze body resources from HRV and ACC |
| 130 | Calculate time elapsed from last night's sleep |
| 132 | Calculate and provide the current Sleep Pressure and feedback |

104

Anaylsis process for The Sleep Pressure

# Fig. 1

Fig. 2a

# An example of sleep pressure calculation in real -time

Starting value without measurement history:
Person is 32 years ? baseline sleep need 474 min (7h 54 min)

Sleep pressure change per each minute awake = 100/(1440-474) = +0.1035
Sleep pressure change per each minute asleep = -100/474 = -0.211

History values

Sleep need is checked 1-2 times a day and real-time analysis is updated accordingly

| | Time of day | Awake or Sleep minutes elapsed | Body resources | Body resources modified to "sleep pressure scale" | Initial sleep pressure that changes with elapsed time | Adjusted final sleep pressure for feedback |
|---|---|---|---|---|---|---|
| Awake | 7 AM | 0 | 80 (starting value for body resources according to time of day, when the device attached) | 20 | 10 (starting value for sleep pressure according to time of day, when the device attached) | 15 |
| | 12 AM | 300 | 54 | 46 | 41 | 44 |
| | 2 PM | 420 | 45 | 55 | 53 | 54 |
| Sleep | 9 PM | 780 | 27 | 73 | 81 | 77 |
| | 11 PM | 900 | 22 | 78 | 93 | 86 |
| | 6 AM | 420 (sleep) | 93 | 7 | 5 | 6 |

Fig. 2b

EP 3 677 171 B1

Fig. 3a) Stress history

Fig. 3b) Short term weekly training load (WTL)

Fig. 3c) Long term training load (MTL)

Fig. 3d) Sleep score

Fig. 3e) Absolute weekly training load

400

402

**Sleep need**

# 7:45

Optimal bedtime at
10:30–11:30 pm

⏰ 06:45 am

404

**Sleep need**

**Updated** ⓘ

Due to your stressful and
busy days your sleep
need has risen. Optimal
bedtime is now 30 min
earlier.

406

**Sleep pressure**

## Low

Last night's restorative sleep
has relieved your sleep
pressure

408

**Sleep pressure**

## High

Earlier bedtime suggested to
ensure restorative sleep

🏃 Vigorous exercise

🌙 Poor quality sleep

# Fig. 4

EP 3 677 171 B1

| | | Sleep duration (hours) | | | | | | | | | | | | |
|---|---|---|---|---|---|---|---|---|---|---|---|---|---|---|
| | | Good | Good | Good | Mod | Mod | Mod | Poor | Poor | Poor | Poor | Poor | Poor | Poor |
| **Sleep quality points** | | *8* | *7.5* | *7* | *6.5* | *6* | *5.5* | *5* | *4.5* | *4* | *3.5* | *3* | *2.5* | *2* |
| | *points* | 100 | 85 | 70 | 57 | 43 | 30 | 26 | 21 | 17 | 13 | 9 | 4 | 0 |
| Good | 100 | 100 | 94 | 87 | 81 | 76 | 70 | 57 | 45 | 34 | 28 | 13 | 3 | 0 |
| Good | 90 | 94 | 88 | 81 | 76 | 70 | 64 | 52 | 41 | 31 | 21 | 11 | 3 | 0 |
| Good | 80 | 89 | 82 | 76 | 70 | 64 | 59 | 48 | 37 | 28 | 19 | 10 | 2 | 0 |
| Good | **70** | 83 | 76 | 70 | 64 | 59 | 53 | 43 | 34 | 25 | 17 | 9 | 2 | 0 |
| Moderate | 60 | 77 | 71 | 64 | 59 | 53 | 47 | 38 | 30 | 22 | 15 | 8 | 2 | 0 |
| Moderate | 50 | 72 | 65 | 59 | 53 | 47 | 41 | 33 | 26 | 19 | 12 | 7 | 1 | 0 |
| Moderate | 40 | 66 | 59 | 53 | 47 | 41 | 36 | 28 | 22 | 16 | 10 | 5 | 1 | 0 |
| Moderate | **30** | 60 | 54 | 47 | 41 | 36 | 30 | 24 | 18 | 13 | 8 | 4 | 1 | 0 |
| Poor | 20 | 47 | 42 | 36 | 31 | 26 | 21 | 16 | 12 | 9 | 5 | 3 | 1 | 0 |
| Poor | 10 | 26 | 23 | 19 | 16 | 13 | 10 | 8 | 5 | 4 | 2 | 1 | 0 | 0 |
| Poor | 0 | 9 | 7 | 6 | 5 | 4 | 3 | 2 | 1 | 1 | 0 | 0 | 0 | 0 |

## Fig. 5

Sleep duration change (24h RSI)

$y = 0.0103x^2 - 1.24x - 0.85/1$

Fig. 6

Fig.7

Audio

75

Voice synt.

Display

14

63 63'

61B

63

RAM

61A

ETE
ROM

62

CPU

61

Input
unit

BUS  66

Output
unit

64

Interface

67

68

BT
WiFi
Mobile data

Keys

78

72

Sensor 1
(Heart rate)

Sensors:

Altitude

GPS

70

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8468115 B
- US 10325514 B
- US 2016374567 A1
- US 2018085549 A1
- US 2015116117 A1